# EUROPEAN PATENT SPECIFICATION

(11) **EP 1 575 633 B1**
(45) Date of publication and mention of the grant of the patent: **17.06.2009**
(21) Application number: 03813580.2
(22) Date of filing: 18.12.2003
(51) Int. Cl.: A61L 27/34, A61L 31/10, G02B 1/04

(54) **MEDICAL DEVICES HAVING ANTIMICROBIAL COATINGS THEREON**
MEDIZINPRODUKTE MIT ANTIMIKROBIELLEN BESCHICHTUNGEN
DISPOSITIFS MEDICAUX POURVUS DE REVETEMENTS ANTIMICROBIENS

(30) Priority: 19.12.2002 US 435003 P
(43) Date of publication of application: 21.09.2005
(73) Proprietor: NOVARTIS AG, 4056 Basel (CH)
(72) Inventor: QIU, Yongxing, Duluth, GA 30097 (US); WINTERTON, Lynn, Cook, Alpharetta, GA 30201 (US); LALLY, John, Martin, Lilburn, GA 30047 (US); KOTOV, Nicholas, Stillwater, OK 74074 (US)
(74) Representative: Bohest AG
(86) International application number: PCT/EP2003/014533
(87) International publication number: WO 2004/056404

(56) References cited:
- WO-A-01/57118
- WO-A-01/92924
- WO-A-99/35520
- WO-A-02/097481
- US-A1- 2001 045 676
- KOKTYSH, DMITRY S. ET AL: "Biomaterials by design: Layer-by-layer assembled ion-selective and biocompatible films of TiO2 nanoshells for neurochemical monitoring" ADVANCED FUNCTIONAL MATERIALS (2002), 12(4), 255-265, November 2002 (2002-11), XP002235084
- ZHANG, JUNHU ET AL: "Thin Films of Ag Nanoparticles Prepared from the Reduction of AgI Nanoparticles in Self-Assembled Films" JOURNAL OF COLLOID AND INTERFACE SCIENCE (2002), 255(1), 115-118, 2002, XP001149415
- WANG, TOM C. ET AL: "Metallodielectric photonic structures based on polyelectrolyte multilayers" ADVANCED MATERIALS (WEINHEIM, GERMANY) (2002), 14(21), 1534-1537, 2002, XP002235085

## Description

The present invention generally relates to a medical device having an antimicrobial metal-containing layer-by-layer coating thereon and to a method for making the medical device of the invention.

### BACKGROUND

Contact lenses are often exposed to one or more microorganisms during wear, storage and handling. They can provide surfaces onto which the microorganisms can adhere and then proliferate to form a colony. Microbial adherence to and colonization of contact lenses may enable microorganisms to proliferate and to be retained at the ocular surface for prolonged periods and thereby may cause infection or other deleterious effects on the ocular health of the eye in which the lens is used. Therefore, it is desirous to make various efforts to minimize and/or eliminate the potential for microorganism adhesion to and colonization of contact lenses.

Many attempts have been made to develop antimicrobial medical devices. Two approaches have been proposed. One approach is to incorporate antimicrobial compounds into a polymeric composition for molding a contact lens. For example, Chalkley et al. in Am. J. Ophthalmology 1968, 61:866-869, disclosed that germicidal agents were incorporated into contact lenses. U.S. Pat. No. 4,472,327 discloses that antimicrobial agents may be added to the monomer before polymerization and locked into the polymeric structure of the lens. U.S. Pat. Nos. 5,358,688 and 5,536,861 disclose that contact lenses having antimicrobial properties may be made from quaternary ammonium group containing organosilicone polymers. European patent application EP 0 604 369 discloses that deposit-resistant contact lenses can be prepared from hydrophilic copolymers that are based on 2-hydroxyethyl methacrylate and comonomers containing a quaternary ammonium moiety. Another example is an ocular lens material, disclosed in European patent application EP 0 947 856 A2, which comprises a quaternary phosphonium group-containing polymer. A further example is U.S. Pat. No. 5,515,117 which discloses contact lenses and contact lens cases made from materials which comprise polymeric materials and effective antimicrobial components. A still further example Is U.S. Pat. No. 5,213,801 which discloses contact lenses made from materials comprising a hydrogel and an antimicrobial ceramic containing at least one metal selected from Ag, Cu and Zn. There are some disadvantages associated with this approach for making antimicrobial contact lenses. Polymeric compositions having antimicrobial properties may not possess all properties desired for contact lenses, especially extended-wear contact lenses, which hinders their practice uses.

The other approach for making antimicrobial medical devices is to form antimicrobial coatings, containing leachable or covalently attached antimicrobial agents, on medical devices. Antimicrobial coatings containing leachable antimicrobial agents may not be able to provide antimicrobial activity over the period of time when used in the area of the human body. In contrast, antimicrobial coating containing covalently bound antimicrobial agents can provide antimicrobial activity over a relatively longer period of time. However, antimicrobial compounds in such coatings may exhibit diminished activity when comparing the activity of the unbound corresponding antimicrobial compounds In solution, unless assisted by hydrolytic breakdown of either the bound antimicrobial compounds or the coating itself. Like the above-described approach, the antimicrobial coating may not be able to provide desired surface properties such as hydrophilicity and/or lubricity and also may have adverse effects on the desired bulk properties of a medical device (for example, the oxygen permeability of a contact lens).

Currently, a wide variety of antimicrobial agents have been proposed to be used as coatings for contact lenses (see, for example, U.S. Pat. No. 5,328,954), Prior known antimicrobial coatings include antibiotics, lactoferrin, metal chelating agents, substituted and unsubstituted polyhydric phenols, amino phenols, alcohols, acid and amine derivatives, and quaternary ammonium group-containing compounds. However, such antimicrobial coatings have disadvantages and are unsatisfactory. The overuse of antibiotics can lead to proliferation of antibiotic-resistant microorganisms. Other coatings may not have broad spectrum antimicrobial activity, may produce ocular toxicity or allergic reactions, or may adversely affect lens properties required for ensuring corneal health and for providing the patient with good vision and comfort.

Therefore, there is a need for antimicrobial coatings that can provide high bactericidal efficacy and broad spectrum antimicrobial activity coupled with low cytotoxicity. There is also a need for new contact lenses having antimicrobial coatings, which have high bactericidal efficacy, a broad spectrum of antimicrobial activities, and minimal adverse effects on the wearer's ocular health and comfort. Such contact lenses may have increased safety as extended-wear contact lenses which could provide comfort, convenience, and safety.

Moreover, surgical and device related infection remains to be one of the main clinical and economic challenges in the field of medical devices and In heath care industry in general. Each year, as many as 2 million hospital patients in the United States develop nosocomial infections, and approximately 80% of the 80,000 annual deaths due to nosocomial infections are device-related. A potent and cost-effective antimicrobial coating for medical devices would be a key to mitigate the infection-related clinical challenges and economic burden of health care.

One object of the invention is to provide an antimicrobial coating which has a high antimicrobial efficacy coupled with low cytotoxicity.

Another object of the invention is to provide a medical device having an antimicrobial coating that has a high Antimicrobial efficacy coupled with low cytotoxicity.

A further object of the invention is to provide a cost-effective and efficient process for forming an antimicrobial coating on a medical device.

### SUMMARY OF THE INVENTION

These and other objects of the invention are met by the various aspects of the invention described herein.

The invention, in one aspect, provides a medical device having a core material and an antimicrobial metal-containing layer-by-layer (LbL) coating that is not covalently attached to the medical device and can impart to the medical device an increased hydrophilicity, characterized by having an averaged contact angle of less than 80 degrees. The antimicrobial metal-containing LbL coating comprises a member selected from the group consisting of: (a) one layer of charged antimicrobial metal nanoparticles; (b) one layer of charged antimicrobial metal-containing nano-particles: (c) silver-polyelectrolyte complexes formed between silver ions and a polycationic material having amino groups; (d) silver-polyelectrolyte complexes formed between silver ions and a polyionic material having sulfur-containing groups: (e) sliver nano-particles; and (f) combinations thereof.

The invention, In another aspect, provides a method for preparing a medical device having an Antimicrobial metal-containing LbL coating thereon comprising the steps of:
(a) contacting said medical device with a solution of a first charged material to form a layer of the first charged material on the medical device;
(b) optionally rinsing said medical device by contacting said medical device with a first rinsing solution;
(c) contacting said medical device with a solution of a second charged material to form a layer of the second charged material on top of the layer of the first charged material, wherein the second charged material has charges opposite of the charges of the first charged material; and
(d) optionally rinsing said medical device by contacting said medical device with a second rinsing solution,
wherein at least one of the first and second charged material is selected from the group consisting of charged antimicrobial metal nanoparticles, charged antimicrobial metal-containing nano-particles, silver-polyelectrolyte complexes formed between silver ions and a polycationic material having amino groups, silver-polyelectrolyte complexes formed between silver ions and a polyionic material having sulfur-containing groups, and combinations thereof..

The invention, in a further aspect, relates to a method for preparing a medical device having an Antimicrobial metal-containing LbL coating thereon, comprising the steps of dipping said medical device in a solution containing a first charged material and a second charged material for a desired period of time so as to obtain the antimicrobial LbL coating characterized by having an average contact angle of 80 degrees or less, wherein the second charged material has charges opposite of the charges of the first charged material, wherein the first charged material and the second charged material are present in an amount such that the ratio of the charges of the first charged material to the second charged material is from 3:1 to 100:1, wherein at least one of the first and second charged material is selected from the group consisting of charged antimicrobial metal nanoparticles, charged antimicrobial metal-containing nano-particles, silver-polyelectrolyte complexes formed between silver ions and a polycationic material having amino groups, silver-polyelectrolyte complexes formed between silver ions and a polyionic material having sulfur-containing groups, and combinations thereof.
In still anoter aspect, the invention relates to a method for producing a contact lens having an antimicrobial metal-containing LbL coating thereon, comprising the steps of:
(a) forming a mold for making the contact lens, wherein the mold comprises a first mold portion defining a first optical surface and a second mold portion defining a second optical surface, wherein said first mold portion and said second mold portion are configured to receive each other such that a contact lens-forming cavity is formed between said first optical surface and said second optical surface;
(b) applying a transferable antimicrobial LbL coating, using a layer-by-layer polyelectrolyte deposition technique, onto at least one of said optical surface, wherein the transferable antimicrobial LbL coating comprises at least one layer of a first charged material and at least one layer of a second charged material having charges opposite of the charges of the first charged material, wherein at least one of the first and second charged material is selected from the group consisting of charged antimicrobial metal nano-particles, charged antimicrobial metal-containing nanoparticles, silver-polyelectrolyte complexes formed between silver ions and a polycationic material having amino groups, silver-polyelectrolyte complexes formed between silver ions and a polyionic material having sulfur-containing groups, and combinations thereof;
(c) positioning said first mold portion and said second mold portion such that said mold portions receive each other and said optical surfaces define said contact lens forming cavity; (d) dispensing a polymerizable composition into said contact lens-forming cavity; and (e) curing said polymerizable composition within said contact lons-forming cavity such that the contact lens is formed, whereby said transferable antimicrobial LbL coating detaches from said at least one optical surface of said mold portion and reattaches to said formed contact lens such that said contact lens becomes coated with the antimicrobial metal-containing LbL coating having an averaged contact angle of less than 80 degrees.

These and other aspects of the invention will become apparent from the following description of the presently preferred embodiments.

### DETAILED DESCRIPTION OF THE PREFERRED EMBODIMENTS

Unless defined otherwise, all technical and scientific terms used herein have the same meaning as commonly understood by one of ordinary skill in the art to which this invention belongs. Generally, the nomenclature used herein and the laboratory procedures are well known and commonly employed in the art. Conventional methods are used for these procedures, such as those provided in the art and various general references. Where a term Is provided in the singular, the Inventors also contemplate the plural of that term. The nomenclature used herein and the laboratory procedures described below are those well known and commonly employed in the art. As employed throughout the disclosure, the following terms, unless otherwise Indicated, shall be understood to have the following meanings.

An "article" refers to an ophthalmic lens, a mold for making an ophthalmic lens, or a medical device other than ophthalmic lens.

A "medical device", as used herein, refers to a device or a part thereof having one or more surfaces that contact tissue, blood, or other bodily fluids of patients in the course of their operation or utility. Exemplary medical devices include: (1) extracorporeal devices for use in surgery such as blood oxygenators, blood pumps, blood sensors, tubing used to carry blood and the like which contact blood which is then returned to the patient; (2) prostheses implanted in a human or animal body such as vascular grafts, stents, pacemaker leads, heart valves, and the like that are implanted in blood vessels or in the heart; (3) devices for temporary intravascular use such as catheters, guide wires, and the like which are placed into blood vessels or the heart for purposes of monitoring or repair; (4) artificial tissues such as artificial skin for burn patients; (5) dentifices, dental moldings; (6) ophthalmic devices; and (7) cases or containers for storing ophthalmic devices or ophthalmic solutions.

An "ophthalmic device", as used herein, refers to a contact lens (hard or soft), an intraocular lens, a corneal onlay, other ophthalmic devices (e.g., stents, glaucoma shunt, or the like) used on or about the eye or ocular vicinity.

"Biocompatible", as used herein, refers to a material or surface of a material, which may be in intimate contact with tissue, blood, or other bodily fluids of a patient for an extended period of time without significantly damaging the ocular environment and without significant user discomfort.

"Ophthalmically compatible", as used herein, refers to a material or surface of a material which may be in intimate contact with the ocular environment for an extended period of time without significantly damaging the ocular environment and without significant user discomfort, Thus, an ophthalmically compatible contact lens will not produce significant corneal swelling, will adequately move on the eye with blinking to promote adequate tear exchange, will not have substantial amounts of protein or lipid adsorption, and will not cause substantial wearer discomfort during the prescribed period of wear.

"Ocular environment", as used herein, refers to ocular fluids (e.g., tear fluid) and ocular tissue (e.g., the cornea) which may come into intimate contact with a contact lens used for vision correction, drug delivery, wound healing, eye color modification, or other ophthalmic applications.

A "monomer" means a low molecular weight compound that can be polymerized. Low molecular weight typically means average molecular Weights less than 700 Daltons,

A "macromer" refers to medium and high molecular weight compounds or polymers that contain functional groups capable of further polymerization. Medium and high molecular weight typically means average molecular weights greater than 700 Daltons.

"Polymer" means a material formed by polymerising one or more monomers.

"Surface modification", as used herein, means that an article has been treated in a surface treatment process (or a surface modification process), in which, by means of contact with a vapor or liquid, and/or by means of application of an energy source (1) a coating is applied to the surface of an article, (2) chemical species are adsorbed onto the surface of an article, (3) the chemical nature (e.g., electrostatic charge) of chemical groups on the surface of an article are altered, or (4) the surface properties of an article are otherwise modified.

"LbL coating", as used herein, refers to a coating that is not covalently attached to an article, preferably a medical device, and is obtained through a layer-by-layer ("LbL") deposition of polyionic or charged materials on an article.

The term "bilayer" is employed herein In a broad sense and is Intended to encompass: a coating structure formed on a medical device by alternatively applying, in no particular order, one layer of a first polyionic material (or charged material) and subsequently one layer of a second polyionic material (or charged material) having charges opposite of the charges of the first polyionic material (or the charged material); or a coating structure formed on a medical device by alternatively applying, in no particular order, one layer of a first charged polymeric material and one layer of a non-charged polymeric material or a second charged polymeric material. It should be understood that the layers of the first and second coating materials (described above) may be intertwined with each other in the bilayer.

A medical device having a core material and an LbL coating, which comprises at least one layer of a charged polymeric material and one layer of a non-charged polymeric material that can be non-covalently bonded to the charged polymeric material, can be prepared according to a method disclosed comprising the steps of (a) contacting the medical device with a solution of a charged polymeric material to form a layer of the charged polymeric material; (b) optionally rinsing the medical device by contacting the medical device with a rinsing solution;
(c) contacting the medical device with a solution of a non-charged polymeric material to form a layer of the non-charged polymeric material on top of the layer of the charged polymeric material, wherein the non-charged polymeric material is capable of being non-covalently bond to the charged polymeric material; and
(d) optionally rinsing the medical device by contacting the medical device with a rinsing solution.

As used herein, "asymmetrical coatings" on an ophthalmic lens refers to the different coatings on the first surface and the opposite second surface of the ophthalmic lens. As used herein, "different coatings" refers to two coatings that have different surface properties or functionalities.

A "capping layer", as used herein, refers to the last layer of a coating material which is applied onto the surface of a medical device.

A "capping bilayer", as used herein, refers to the last bilayer of a first coating material and a second coating material, which is applied onto the surface of a medical device.

A "polyquat", as used herein, refers to a polymeric quaternary ammonium group-containing compound.

As used herein, a "polyionic material" refers to a polymeric material that has a plurality of charged groups, such as polyelectrolytes, p- and n-type doped conducting polymers. Polyionic materials include both polycationic (having positive charges) and polyanionic (having negative charges) materials.

An "antimicrobial LbL coating", as used herein, refers to an LbL coating that imparts to a medical device the ability to decrease or eliminate or inhibit the growth of microorganisms on the surface of the medical device or in an adjacent area extending from the medical device. An antimicrobial LbL coating on a medical device of the invention exhibit preferably at least a 1-log reduction (≥90% inhibition), more preferably at least a 2-log reduction (≥99% inhibition), of viable microorganisms.

An "antimicrobial agent", as used herein, refers to a chemical that is capable of decreasing or eliminating or inhibiting the growth of microorganisms such as that term is known In the art.

"Antimicrobial metals" are metals whose ions have an antimicrobial effect and which are biocompatible, Preferred antimicrobial metals include Ag, Au, Pt, Pd, Ir, Sn, Cu, Sb, Bi and Zn, with Ag being most preferred.

"Antimicrobial metal-containing nanoparticles" refers to particles having a size of less than 1 micrometer and containing at least one antimicrobial metal present in one or more of its oxidation states. For example, silver-containing nanoparticles can contain silver in one or more of its oxidation states, such as Ag⁰, Ag¹⁺, and Ag²⁺.

"Antimicrobial metal nanoparticles" refers to particles which is made of one or more antimicrobial metals and have a size of less than 1 micrometer. The antimicrobial metals in the antimicrobial metal nanoparticles can be present in one or more of its oxidation state.

An "averaged contact anglo " refers to a contact angle (Sessile Drop), which is obtained by averaging measurements of at least 3 individual medical devices.

As used herein, "increased surface hydrophilicity" or "increased hydrophilicity" in reference to a coated medical device means that the coated medical device has a reduced averaged contact angle compared with an uncoated medical device.

The present invention is directed to a medical device having a core material and an antimicrobial metal-containing LbL surface coating (hereinafter LbL coating) formed thereon and to a method for making the same. The antimicrobial metal-containing LbL coating imparts to the medical device an increased surface hydrophilicity (hereinafter hydrophilicity) and exhibits at least 50% inhibition of viable microorganisms. The increased hydrophilicity is characterized by having an averaged contact angle of 80 degrees or less.

It has been discovered here that an antimicrobial metal, silver, In particular silver nano-particles, and silver-polyelectrolyte complexes can be incorporated cost-effectively into an LbL coating according to one of the methods of the invention. It is found that an silver-containing LbL coating of the invention may possess several advantages as follows. It can impart to a medical device not only an antimicrobial activity but also an increased surface hydrophilicity. It has minimal adverse effects on the desired bulk properties of, for example, a contact lens, such as oxygen permeability, ion permeability, and optical properties. An silver-containing LbL coating of the invention formed on a medical device can adhere well to a medical device and be stable, even after several cycles of autoclaving treatments, The process for forming a silver-containing LbL coating of the invention is well suited for automation and can be used to coat a wide range of substrate (polymeric, glass, quartz, ceramic, metal) and in any geometry. Out-diffusion of silver from the silver-containing coating is controllable. In addition, a medical device having an antimicrobial LbL coating of the invention thereon can be further subjected to surface modification, such as plasma treatment to obtain a coating possessing advantages of both plasma coating and an antimicrobial coating of the invention.

In accordance with the present invention, the core material of a medical device (substrate) may be any of a wide variety of polymeric materials. Exemplary core materials include, but are not limited to, hydrogels, silicone-containing hydrogels, polymers and copolymers of styrene, substituted styrenes, ethylene, propylene, acrylates, methacrylates. N-vinyl lactams, acrylamides and methacrylamides, acrylonitrile, acrylic acid, methacrylic acid, or combinations thereof.

A preferred group of core materials to be coated are those being conventionally used for the manufacture of biomedical devices, e.g. contact lenses, in particular contact lenses for extended wear, which are not hydrophilic per se. Such materials are known to the skilled artisan and may comprise for example polysiloxanes, perfluoroalkyl polyethers, fluorinated poly(meth)acrylates or equivalent fluorinated polymers derived e.g. from other polymerizable carboxylic acids, polyalkyl (meth)acrylates or equivalent alkylester polymers derived from other polymerizable carboxylic acids, or fluorinated polyolefins, such as fluorinated ethylene or propylene, for example tetrafluoroethylene, preferably in combination with specific dioxols, such as perfluoro-2,2-dimethyl-1,3-dioxol. Examples of suitable bulk materials are e.g. Lotraflicon A, Neofocon, Pasifocon, Telefocon, Silafocon, Fluorsilfocon, Paflufocon, Silafocon, Elastofilcon, Balifilcon A, Fluorofocon, or Teflon AF materials, such as Teflon AF 1600 or Teflon AF 2400 which are copolymers of 63 to 73 mol % of perfluoro-2,2-dimethyl-1,3-dioxol and 37 to 27 mol % of tetrafluoroethylene, or of 80 to 90 mol % of perfluoro-2,2-dimethyl-1,3-dioxol and 20 to 10 mol % of tetrafluoroethylene.

The core material underlying the composite materials of the invention is preferably a material that is devoid of ionic groups such as cationic or anionic groups. Accordingly, the surface of the preferred core materials is also devoid of ionic groups such as carboxy, sulfo, amino and the like groups and is thus substantially free from ionic charges.

Another group of preferred core materials to be coated is amphiphilic-segmented copolymers comprising at least one hydrophobic segment and at least one hydrophilic segment, which are linked through a bond or a bridge member. Examples are silicone hydrogels, for example those disclosed in PCT applications WO 96/31792 to Nicolson et al. and WO 97/49740 to Hirt et al.

A particular preferred group of core materials to be coated comprises organic polymers selected from polyacrylates, polymethacrylates, polyacrylamides, poly(N,N-dimethylacrylamides), polymethacrylamides, polyvinyl acetates, polysiloxanes, perfluoroalkyl polyethers, fluorinated polyacrylates or -methacrylates and amphiphilic segmented copolymers comprising at least one hydrophobic segment, for example a polysiloxane or perfluoroalkyl polyether segment or a mixed polysiloxane/perfluoroalkyl polyether segment, and at least one hydrophilic segment, for example a polyoxazoline, poly(2-hydroxyethyl-methacrylate), polyacrylamide, poly(N,N-dimethylacrylamide), polyvinylpyrrolidone polyacrylic or polymethacrylic acid segment or a copolymeric mixture of two or more of the underlying monomers.

The core material to be coated may also be any blood-contacting material conventionally used for the manufacture of renal dialysis membranes, blood storage bags, pacemaker leads or vascular grafts. For example, the material to be modified on its surface may be a polyurethane, polydimethylsiloxane, polytetrafluoroethylene, polyvinylchloride, Dacron^{™} or Silastio^{™} type polymer, or a composite made therefrom.

Moreover, the core material to be coated may also be an Inorganic or metallic base material without suitable reactive groups, e.g. ceramic, quartz, or metals, such as silicon or gold, or other polymeric or non-polymeric substrates, e.g., for implantable biomedical applications, ceramics are very useful. In addition, e.g. for biosensor purposes, hydrophilically coated base materials are expected to reduce nonspecific binding effects if the structure of the coating is well controlled. Biosensors may require a specific carbohydrate coating on gold, quartz, or other non-polymeric substrates.

The core material to be coated can be subjected to a surface modification before applying an antimicrobial coating. Exemplary surface treatment processes include, but are not limited to, a surface treatment by energy (e.g., a plasma, a static electrical charge, irradiation, or other energy source), chemical treatments, the grafting of hydrophilic monomers or macromers onto the surface of an article, and layer-by-layer deposition of polyelectrolytes. A preferred class of surface treatment processes are plasma processes, in which an ionized gas is applied to the surface of an article. Plasma gases and processing conditions are described more fully in U.S. Pat. Nos. 4,312,575 and 4,632,844. The plasma gas is preferably a mixture of lower alkanes and nitrogen, oxygen or an inert gas.

The form of the core material to be coated may vary within wide limits. Examples are particles, granules, capsules, fibers, tubes, films or membranes, preferably moldings of all kinds such as ophthalmic moldings, for example intraocular lenses, artificial cornea or in particular contact lenses.

Coating materials for forming an antimicrobial metal-containing LbL coating include, without limitation, polyionic materials, non-charged polymeric materials, polymerized vesicles (liposomes and micelles) with surface charges, charged antimicrobial metal nanoparticles (preferably charged silver nano-particles), charged antimicrobial metal-containing nanoparticles (preferably charged silver-containing nanoparticles), silver-polyelectrolyte complexes formed between silver ions and a polyionic material having sulfur-containing groups, silver-polyelectrolyte complexes formed between silver ions and a polycationic material having amino groups, a negatively charged polyionic material having -COOAg groups, and combinations thereof.

The polyionic materials that may be employed in the present invention include polyanionic and polycationic polymers. Examples of suitable polyanionic polymers include, for example, a synthetic polymer, a biopolymer or modified biopolymer comprising carboxy, sulfo, sulfato, phosphono or phosphato groups or a mixture thereof, or a salt thereof, for example, a biomedical acceptable salt and especially an ophthalmically acceptable salt thereof when the article to be coated is an ophthalmic device.

Examples of synthetic polyanionic polymers are: a linear polyacrylic acid (PAA), a branched polyacrylic acid, a polymethacrylic acid (PMA), a polyacrylic acid or polymethacrylic acid copolymer, a maleic or fumaric acid copolymer, a poly(styrenesulfonic acid) (PSS), a polyamido acid, a carboxy-terminated polymer of a diamine and a di- or polycarboxylic acid (e.g., carboxy-terminated Starburst^{™} PAMAM dendrimers from Aldrich), a poly(2-acrylamido-2-methylpropanesulfonic acid) (poly-(AMPS)), an alkylene polyphosphate, an alkylene polyphosphonate, a carbohydrate polyphosphate or carbohydrate polyphosphonate (e.g., a teichoic acid). Examples of a branched polyacrylic acid include a Carbophil^{®} or Carbopol^{®} type from Goodrich Corp. Examples of a copolymer of acrylic or methacrylic acid include a copolymerization product of an acrylic or methacrylic acid with a vinyl monomer including, for example, acrylamide, N,N-dimethyl acrylamide or N-vinylpyrrolidone.

Examples of polyanionic biopolymers or modified biopolymers are: hyaluronic acid, glycosaminoglycanes such as heparin or chondroitin sulfate, fucoidan, poly-aspartic acid, poly-glutamic acid, carboxymethyl cellulose, carboxymethyl dextrans, alginates, pectins, gellan, carboxyalkyl chitins, carboxymethyl chitosans, sulfated polysaccharides.

A preferred polyanionic polymer is a linear or branched polyacrylic acid or an acrylic acid copolymer. A more preferred anionic polymer is a linear or branched polyacrylic acid. A branched polyacrylic acid in this context is to be understood as meaning a polyacrylic acid obtainable by polymerizing acrylic acid in the presence of suitable (minor) amounts of a di- or polyvinyl compound.

A suitable polycationic polymer as part of the bilayer is, for example, a synthetic polymer, biopolymer or modified biopolymer comprising primary, secondary or tertiary amino groups or a suitable salt thereof, preferably an ophthalmically acceptable salt thereof, for example a hydrohalogenide such as a hydrochloride thereof, in the backbone or as substituents. Polycationic polymers comprising primary or secondary amino groups or a salt thereof are preferred.

Examples of synthetic polycationic polymers are:
(i) a polyallylamine (PAH) homo- or copolymer, optionally comprising modifier units;
(ii) a polyethyleneimine (PEI);
(iii) a polyvinylamine homo- or copolymer, optionally comprising modifier units;
(iv) a poly(vinylbenzyl-tri-C₁-C₄-alkylommonium Salt), for example a poly(vinylbenzyl-trimethyl ammoniumchloride);
(v) a polymer of an aliphatic or araliphatic dihalide and an aliphatic N,N,N',N'-tetra-C₁-C₄. alkyl-alkylenedlemine, for example a polymer of (a) propyfene-1,3-dlchlorlde or - dibromide or p-xylylene dichloride or dibromide and (b) N,N,N',N'-tetramethyl-1,4-tetramethylene diamine;
(vi) a poly(vinylpyridine) or poly(vinylpyridinium salt) homo- or copolymer;
(vii) a poly(N,N-diallyl-N,N-di-C₁-C₄-alkyl-ammoniumhalide);
(viii) a homo- or copolymer of a quaternized di-C₁-C₄-alkyl-aminoethyl acrylate or methacrylate, for example a poly(2-hydroxy-3-methacryloylpropyltri-C₁-C₂-alkylammonium salt) homopolymer such as a a poly(2-hydroxy-3-methacryloylpropyltri-methylammonium chloride), or a quaternized poly(2-dimethylaminoethyl methacrylate or a quaternized poly(vinylpyrrolidone-co-2-dimethylaminoethyl methacrylate);
(ix) polyquat; or
(x) a polyaminoamide (PAMAM), for example a linear PAMAM or a PAMAM dendrimer such as an amino-terminated Starbust^{™} PAMAM dendrimer (Aldrich).

The above mentioned polymers comprise in each case the free amine, a suitable salt thereof, for example a biomedically acceptable saft or in particular an ophthalmically acceptable salt thereof, as well as any quaternized form, if not specified otherwise.

Suitable comonomers optionally incorporated In the polymers according to (i), (iii), (vi) or (viii) above are, for example, hydrophilic monomers such as acrylamide, methacrylamide, N,N-dimethyl acrylamide, N-vinylpyrrolidone and the like.

Examples of polycationic biopolymers or modified biopolymers that may be employed in the bilayer of the present invention include: basic peptides, proteins or glucoproteins, for example, a poly-ε-lysine, albumin or collagen, aminoalkylated polysaccharides such as a chitosan or aminodextranes.

Particular polycationic polymers for forming the bilayer of the present invention include a polyallylamine homopolymer; a polyallylamine comprising modifier units of the above formula (II); a polyvinylamine homo- or -copolymer or a polyethyleneimine homopolymer, in particular a polyallylamine or polyethyleneimine homopolymer, or a poly(vinylamine-co-acrylamid) copolymer.

The foregoing lists are intended to be exemplary, but clearly are not exhaustive. A person skilled in the art, given the disclosure and teaching herein, would be able to select a number of other useful polyionic materials.

It has been discovered previously that one layer of a charged polymeric material and one layer of a non-charged polymeric material, which can be non-covalently bonded to the charged polymeric material can be alternatively deposited onto a substrate to form a biocompatible LbL coating. The non-charged polymeric material according to the invention can be: a homopolymer of a vinyl lactam; a copolymer of at least one vinyl lactam in the presence or in the absence of one or more hydrophilic vinylic comonomers; or mixtures thereof.

The vinyl lactam has a structure of formula (I) 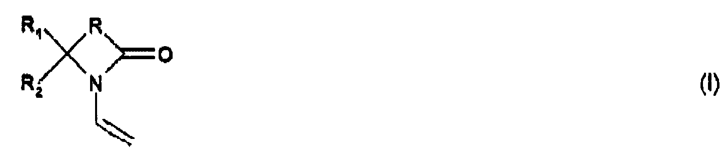 wherein R is an alkylene di-radical having from 2 to 8 carbon atoms. R₁ is hydrogen, alkyl, aryl, aralkyl or alkaryl, preferably hydrogen or lower alkyl having up to 7 and, more preferably, up to 4 carbon atoms, such as, for example, methyl, ethyl or propyl; aryl having up to 10 carbon atoms, and also aralkyl or alkaryl having up to 14 carbon atoms; and R₂ is hydrogen or lower alkyl having up to 7 and, more preferably, up to 4 carbon atoms, such as, for example, methyl, ethyl or propyl.

The invention, in one aspect, provides a medical device having an antimicrobial metal-containing antimicrobial LbL coating that is not covalently attached to the medical device and having an increased hydrophilicity, characterized by having an averaged contact angle of 80 degrees or less.

The antimicrobial LbL coating comprises at least one member selected from the group consisting of: (a) one layer of charged antimicrobial metal nanoparticles; (b) one layer of charged antimicrobial metal-containing nano-particles; (c) silver-polyelectrolyte complexes formed between silver Ions and a polycationic material having amino groups; (d) silver-polyelectrolyte complexes formed between silver ions and a polyionic material having sulfur-containing groups; (e) silver nano-particles; and (f) combinations thereof.

In accordance with a preferred embodiment, the charged antimicrobial metal nanoparticles are silver nanoparticles.

Antimicrobial metal nano-particles can be either positively charged or negatively charged, largely depending on a material (or so-called stabilizer) which is present in a solution for preparing the nano-particles and can stabilize the resultant nano-particles. A stabilizer can be any known suitable material. Exemplary stabilizers include, without limitation, positively charged polyelectrolytes, negatively charged polyelectrolytes, surfactants, salicylic acid, alcohols and the like. Where charged antimicrobial metal nanoparticles are silver nanoparticles, a stabilizer preferably is a chemical with at least one sulfur-containing group. It is known that sulfur binds tightly to silver.

Exemplary sulfur-containing groups include, without limitation, thiol, sulfonyl, sulfonic acid, alkyl sulfide, alkyl disulfide, substituted or unsubstituted phenyldisulfide, thiophenyl, thiourea, thioether, thiazolyl, thiazolinyl, and the like.

Any known suitable methods can be used in the preparation of silver or other antimicrobial metal nano-particles. For example, silver ions or silver salts can be reduced by means of a reducing agent (e.g., NaBH₄ or ascorbic acid or salts thereof) or of heating or UV irradiation in a solution in the presence of a stabilizer to form silver nano-particles. A person skilled in the art will know how to choose a suitable known method for preparing silver nano-particles. Exemplary silver salts include, without limitation, silver nitrate, silver acetate, silver citrate, silver sulfate, silver lactate, and silver halide.

In accordance with the invention, charged antimicrobial metal-containing nanoparticles can comprises at least one antimicrobial metal selected from the group consisting of Ag, Au, Pt, Pd, Ir, Sn, Cu, Sb, Bi and Zn. Preferably, the charged antimicrobial metal-containing nanoparticles are silver-containing nanoparticles.

Any known suitable methods can be used to prepare charged antimicrobial metal-containing nanoparticles. For example, TiO₂ nanoparticles are mixed with AgNO₃ solution to form a mixture, which is subsequently exposed to UV irradiation to coat completely or partially TiO₂ nanoparticles with silver. The TiO₂ nanoparticles having a silver coating thereon can be further coated with one or more polyionic materials by layer-by-layer deposition techniques, to form charged silver-containing nanoparticles.

Alternatively, one or more antimicrobial metals can be coated onto nanoparticles made of any biocompatible materials, by using vapor deposition techniques. Physical vapor deposition techniques, which are well known in the art, all deposit the metal from vapor, generally atom by atom, onto a substrate surface. The techniques include vacuum or arc evaporation, sputtering, magnetronsputtering and ion plating. The nanoparticles having an antimicrobial metal coating thereon can be further coated with one or more polyionic materials by layer-by-layer deposition techniques, to form charged antimicrobial metal-containing nanoparticles.

In another preferred embodiment, the antimicrobial LbL coating of the invention formed on a medical device comprises: at least one layer of charged antimicrobial metal nano-particles or charged antimicrobial metal-containing nanoparticles; and at least one layer of a polyionic material having charges opposite of the charges of the charged antimicrobial metal nano-particles or charged antimicrobial metal-containing nanoparticles.

In another preferred embodiment, the antimicrobial coaling of the invention formed on a medical device comprises: at least one layer of a negatively-charged polyionic material and at least one layer of sliver-polyelectrolyte complexes formed between silver cations and a positively-charged amino group containing polyionic material. Exemplary complexes include, without limitation, complexes formed between silver ions and polyethyleneimine (PEI), complexes formed between silver ions and polyamidoamine (PAMAM) dendrimer, complexes formed between silver ions and a polyquat, and the like.

In another preferred embodiment, the antimicrobial LbL coating of the invention formed on a medical device comprises: at least one layer of silver-polyelectrolyte complexes formed between silver ions and a polyionic material with sulfur-containing groups and at least one layer of a polyionic material having charges opposite of the charges of the polyionic material with sulfur-containing groups,

In another preferred embodiment, the antimicrobial LbL coating of the invention formed on a medical device comprises silver nano-particles which are obtained by first forming a transitional LbL coating composed of at least one layer of a first polyionic material and at least one layer of a second polyionic material having charges opposite of the charges of the first polyionic material, wherein at least one of the first and second polyionic materials is selected from the group consisting of silver-polyelectrolyte complexes formed between silver cations and a positively-charged amino group containing polyionic material, silver-polyelectrolyte complexes formed between silver cations and a polyionic material with sulfur-containing groups, and combinations thereof; and then by reducing silver ions in the transitional LbL coating by means of a reducing agent, W Irradiation or heating.

In a more preferred embodiment, the antimicrobial LbL coating of the invention formed on a medical device comprises at least two members selected from the group consisting of one layer of charged antimicrobial metal nano-particles, one layer of charged antimicrobial metal-containing nanoparticles, one layer of silver-polyelectrolyte complexes formed between silver ions and a polyionic material with sulfur-containing groups, and one layer of silver-polyelectrolyte complexes formed between silver cations and a positively-charged amino group containing polyionic material. Even more preferably, an antimicrobial metal-containing antimicrobial LbL coating further comprises at least one layer of a polyquat which exhibits antimicrobial activity. Such antimicrobial LbL coating of the invention may exhibit antimcirobial synergy of an antimicrobial metal and the polyquat and therefore may possess a higher antimicrobial efficacy and a broader spectrum of antimicrobial activities.

Any polyquats which exhibit antimicrobial activity can be used in the present invention. Exemplary preferred polyquats are polyquats polymeric quaternary ammonium salt compound (polyquat) of formula (II) or (III) 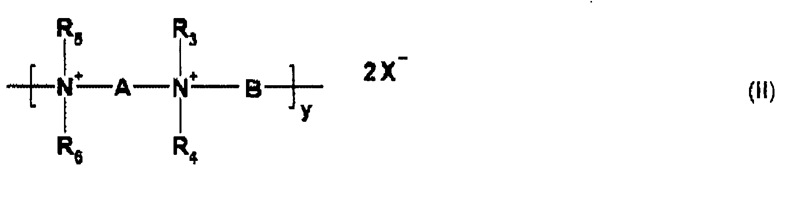 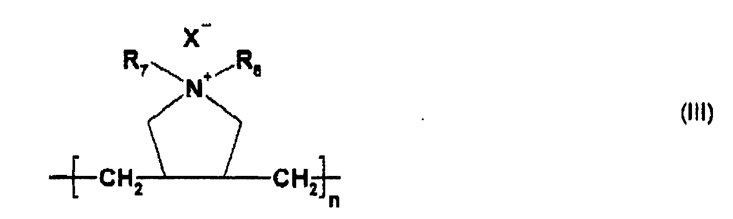 wherein R₃, R₄, R₅ and R₆ independently of each other are C₁-C₁₀ hydrocarbon radicals, preferably C₁ to C₈ alkyl radicals or C₁ to C₆ alkyl radicals having one or more hydroxyl groups; A and B independent of each other are n-alkylene groups having 3 to 15 carbon atoms or n-alkylene groups having 3 to 15 carbon atoms and one or more hydroxyl groups; Y is a number from 10 to 500; n is a number from 100 to 5000; X is chlorine, bromine, or iodine; R₇ and R₈ independently of each other are n-alkyl groups having 1 to 10 carbon atoms or n-alkyl groups having 1 to 10 carbon atoms and one or more hydroxyl groups.

In accordance with a more preferred embodiment of the invention, the surface of a medical device is first coated (completely or partially with at least one antimicrobial agent selected from the group consisting of a polyquat which exhibits antimicrobial activity, furanones, antimicrobial peptides, isoxaxolinones, and organic selenium compounds, and then coated with an antimcirobial metal-containing LbL coating of the invention on top of the coat of the at least one antimicrobial agent. The antimicrobial agents are covalently attached to the surface of the medical device.

In accordance with another more preferred embodiment of the invention, a medical device comprises: an antimicrobial metal-containing LbL coating that is not covalently attached to the medical device; and at least one antimicrobial agent which is covalently attached to the LbL coating through the reactive sites of the LbL coating, wherein said at least one antimicrobial agent is selected from the group consisting of a polyquat which exhibits antimicrobial activity, furanones, antimicrobial peptides, isoxazolinones, and organic selenium compounds,

Such medical device may exhibit antimicrobial synergy of antimicrobial metal and one or more antimicrobial agents and therefore may possess a higher antimicrobial efficacy and a broader spectrum of antimicrobial activities.

Any antimicrobial peptides can be used in the present invention. Exemplary antimicrobial peptides include without limitation Cecropin A melittin hybrid, indolicidin, lactoferricin, Defensin 1, Bactenecin (bovin), Magainin 2, functionally equivalent or superior analogs thereof, mutacin 1140, and mixtures thereof.

Cecropin A-melittin hybride has an amino acid sequence of Lys-Trp-Lys-Leu-Phe-Lys-Lys-Ile-Gly-Ala-Val-Leu-Lys-Val-Leu-COOH (or -NH₂).

Cecropin A is a 37-reside peptide and has an amino acid sequence of Lys-Trp-Lys-Leu-Phe-Lys-Lys-Ile-Glu-Ly8-Val-Gly-Gln-Asn- Ile-Arg-Asp-Gly-Ile-Ile-Lys-Ala-Gly-Pro-Ala-Val-Ala-Val- Val-Gly-Gln-Ala-Thr-Gln-Ile-Ala-Lys-NH₂ (or -COOH)

Cecropin P1 has an amino acid sequence of Ser-Trp-Leu-Ser-Lys-Thr-Ala-Lys-Lys-Leu-Glu-Asn-Ser-Ala- Lys-lys-Arg-Ile-Ser-Glu-Gly-Ile-Ala-Ile-Ala-Ile-Gln-Gly- Gly-Pro-Arg.

Lactoferricin (bovine) has an amino acid sequence of Arg-Arg-Trp-Gln-Trp-Arg-Met-Lys-Lys-Leu-Gly.

Bactenecin (bovine) is a cyclic cationic dodecapeptide isolated from bovine neutrophil granules. It has an amino acid sequence of Arg-Leu-Cys-Arg-Ile-Val-Val-Ile-Arg-Val-Cys-Arg.

Defensin 1 is a endogenous antibiotic peptide (T. Ganz, et al., J. Clin, invest., 76, 1427 (1985), M.E. Selsted, et al., J. Clin. invest., 76, 1436 (1965)., T. Ganz, M.E, Selsted, and R.I. Lehrer, Eur. J. Haematol, 44,1 (1990)) and has an amino acid sequence of Ala-Cys-Tyr-Cys-Arg-Ile-Pro-Ala-Cys-Ile-Ala-Gly-Glu-Arg- Arg-Tyr-Gly-Thr-Cys-Ile-Tyr-Gln-Gly-Arg-Leu-Trp-Ala-Phe- Cys-Cys.
Indolicidin is 13-residue peptide amide and has an amino acid sequence of Ile-Leu-Pro-Trp-Lys-Trp-Pro-Trp-Trp-Pro-Trp-Arg-Arg-NH2 (or -COOH).

Magainin 2 is a hemolytic and antimicrobial peptide (A. Mor et al., Biochemistry, 30, 8824 (1981)) and has an amino acid sequence of Gly-Ile-Gly-Lys-Phe-Leu-His-Ser-Ala-Ly5-Lys-Phe-Gly-Lys- Ala-Phe-Val-Gly-Glu-Ile-Met-Asn-Ser.

"Functionally equivalent or superior analogs" of an antimicrobial peptide refers to derivatives of a native antimicrobial peptide in which one or more amino acid residues have been replaced by a different amino acid (conservative amino acid substitution or others) or deleted or inserted to provide equal or better biological activity (i.e., antimicrobial activity). A functionally equivalent or superior analog can be a substitution analog, a deletion analog, or an addition analog.

A "substitution analog" Is a peptide In which one or more amino acid residues have been replaced by a different amino acid (conservative amino acid substitution or others) to provide equal or better biological activity (i.e., antimicrobial activity). A deletion analog is a peptide in which one or more amino acid residues have been deleted to provide equal or better antimicrobial activity. An addition analog is peptide in which one or more amino acid residues have been inserted to provide equal or better biological activity (i.e., antimicrobial activity). A person skilled in the art will know how to design and prepare a substitution analog, For example, U.S. Patent Nos. 5,792,831 and 5,912,231 describe substitution and deletion analogs of Magainin 2.

Antimicrobial peptides can be obtained from commercial suppliers or can be synthesized according to any known suitable method, for example, using an Applied Biosystems Model 430A peptide synthesizer. It is understood in the art that there are other suitable peptide synthetic devices or that manual peptide synthesis could be carried out to produce the peptides of the present invention. Automated solid phase peptide synthesis is described, e.g., in Stewart et al, (1984) Solid Phase Peptide Synthesis, Pierce Chemical Company, Rockford, Illinois).

It is known to a person skilled In the art that an antimicrobial peptide can be produced by expression In a suitable bacterial or eukaryotic host. Suitable methods for expression are described by Sambrook, et al., (In: Molecular Cloning, A Laboratory Manual, 2nd Edition, Cold Spring Harbor Press, Cold Spring Harbor, New York (1989)), or similar texts.

Any furanones, which exhibit antimicrobial activity, can be used in the present invention. Exemplary preferred furanones are those disclosed In PCT published patent applications WO 01/88090 A1 and WO 01/68091 A1. Those furanones generally have the structure of formula (IV): 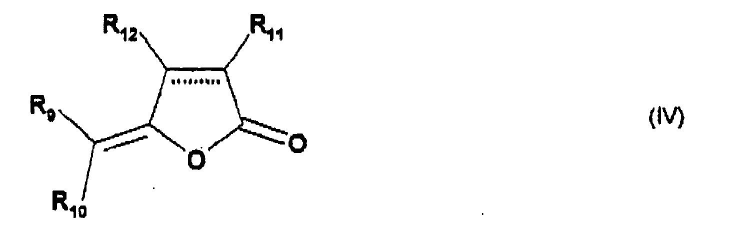 wherein R₁₁ and R₁₂ are independently H, halogen, alkyl, alkoxy, oxoalkyl, alkenyl, aryl or arylalkyl whether unsubstituted or substituted, optionally Interrupted by one or more heteroatoms (i.e., O, N, or S), straight chain or branched chain, hydrophilic or fluorophilic; R₉ and R₁₀ are independently H, halogen, alkyl, aryl or arylalkyl, alkoxy; R₉ or R₁₀ + R₁₂ can be a saturated or an unsaturated cycloalkane; and ( 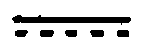 ) represents a single bond or a double bond provided that at least one of R₁₁, R₁₂, R₉ and R₁₀ is halogen. The term "alkyl" used either alone or in compound words preferably denotes a lower alkyl of 1 to carbon atoms.

Any organic selenium compounds, which exhibit an antimicrobial activity, can be used in the present invention, Examples of antimicrobial organic selenium compounds includes without limitation those disclosed in U.S. patent Nos. 5,783,454, 5,994,151, 6,033,917, 6,040,197, 6,043,098, 6,043,099, 6,077,714.

Any isoxazolinones, which exhibit an antimicrobial activity, can be used in the present invention, Examples of isoxazolinones include without limitation those disclosed in US patent Nos. 6,466,456 and 6,420,349 and US patent application Publ. No. 2002/0094984.

An antimicrobial agent can be covalently attached to a medical device by first functionalizing the surface of a preformed medical device to obtain function groups and then covalently attaching an antimicrobial agent. Surface modification (or functionalization) of a medical device is well known to a person skilled in the art, Any known suitable method can be used.

For example, the surface modification of a contact lens includes, without limitation, the grafting of monomers or macromers onto polymers to make the lens biocompatible, wherein monomers or macromers contain functional groups, for example, such as hydroxyl group, amine group, amide group, sulfhydryl group, -COOR (R and R' are hydrogen or C₁ to C₈ alkyl groups), halide (chloride, bromide, iodide), acyl chloride, isothiocyanate, isocyanate, monochlorotriazine, dichlorotriazine, mono- or di-halogen substituted pyridine, mono- or dihalogen substituted diazine, phosphoramidite, maleimide, aziridine, sulfonyl halide, hydroxysuccinimide ester, hydroxysulfosuccinimide ester, imido ester, hydrazine, axidonitrophenyl group, azide, 3-(2-pyridyl dithio)proprionamide, glyoxal, aldehyde, epoxy.

It is well known in the art that a pair of matching functional groups can form a covalent bond or linkage under known reaction conditions, such as, oxidation-reduction conditions, dehydration condensation conditions, addition conditions, substitution (or displacement) conditions, 2+2 cyclo-addition conditions, Diels-Alder reaction conditions. ROMP (Ring Opening Metathesis Polymerization) conditions, vulcanization conditions, cationic crosslinking conditions, and epoxy hardening conditions. For example, an amino group is covalently bondable with aldehyde (Schiff base which is formed from aldehyde group and amino group may further be reduced); an hydroxyl group and an amino group are covalently bondable with carboxyl group; carboxyl group and a sulfo group are covalently bondable with hydroxyl group; a mercapto group is covalently bondable with amino group; or a carbon-carbon double bond Is covalently bondable with another carbon-carbon double bond.

Exemplary covalent bonds or linkage, which are formed between pairs of crosslinkable groups, include without limitation, ester, ether, acetal, ketal, vinyl ether, carbamate, urea, amine, amide, enamine, imine, oxime, amidine, iminoester, carbonate, orthoester, phosphonate, phosphinate, sulfonate, sulfinate, sulfide, sulfate, disulfide, sulfonamide, sulfonamide, thioester, aryl, silane, siloxane, heterocycles, thiocarbonate, thiocarbamate, and phosphonamide.

Another example is amination of the surface of a medical device. If the surface of a core material has hydroxy groups, the medical device may be placed in a bath of an inert solvent, such as tetrahydrofuran, and tresyl chlorine. The hydroxy groups on the surface are then tresylated. Once tresylated, the surface may be aminated in a water solution of ethylene diamine, which results in bonding the group -NH-CH₂-CH₂-NH₂ to the carbon atom thereon. Alternatively, for example, a contact lens made from a hydrogel, can be dipped Into or sprayed with a solution containing a diaziridine compound, which is subsequently attached covalently to the surface of the contact lens via a thermal process, so as to functionalize the contact lens. Such functionalized lenses can be used in covalently attaching of a layer of antimicrobial agents.

A medical device, which comprises an antimicrobial metal-containing LbL coating that is not covalently attached to the medical device and a layer of at least one antimicrobial agent which is covalently attached to the LbL coating through the reactive sites of the LbL coating, can be made by first applying an antimicrobial metal-containing LbL coating to a preformed medical device according to one of the below-described coating methods and then by covalently attaching a layer of at least one antimicrobial agent to some of those reactive sites.

Antimicrobial agents can be bound covalently to the LbL coating. This may be either a direct reaction or, preferably, a reaction in which a coupling agent is used. For example, a direct reaction may be accomplished by the use of a reagent of reaction that activates a group in the LbL coating or the antimicrobial agent making it reactive with a functional group on the antimicrobial agent or LbL coating, respectively, without the incorporation of a coupling agent. For example, one or more amine groups on an LbL coating may be reacted directly with isothiocyanate, acyl azide, N-hydroxysuccinimide ester, sulfonyl chloride, an aldehyde, glyoxal epoxide, 25 carbonate, aryl halide, imido ester, or an anhydride group in an antimicrobial agent.

Alternatively, coupling agents may be used, Coupling agents useful for coupling antimicrobial agent to the LbL coating of a medical device include, without limitation, N. N'-carbonyldiimidazole, carbodiimides such as 1-ethyl-3-(3-dimethylaminopropyl)carbodiimide ('EDC"), dicyclohexyl carbodiimide, 1-cylcohexyl-3-(2-morpholinoethyl)carbodiimide, diisopropyl carbodiimide, or mixtures thereof, The carbodiimides also may be used with N-hydroxysuccinimide or N-hydroxysulfosuccinimide to form esters that can react with amines to form amides.

Amino groups also may be coupled to the LbL coating by the formation of Schiff bases that can be reduced with agents such as sodium cyanoborohydride and the like to form hydrolytically stable amine links. Coupling agents useful for this purpose include, without limitation, N- hydroxysuccinimide esters, such as dithiobis(succinimidylproplonate), 3,3'-dithiobis(sulfosuccinimidylpropionate), disuccinimidyl suberate, bis(sulfosuccinimidyl) suberate, disuccinimidyl tartarate and the like, imidoesters, including, without limitation, dimethyl adipimate, difluorobenzene derivatives, including without limitation 1,5-difluoro-2, 4 dinitrobenzene, bromofunctional aldehydes, including without limitation gluteraldehyde, and his epoxides, including without limitation 1,4- butanediol diglycidyl ether. One ordinarily skilled In the art will recognize that any number of other coupling agents may be used depending on the functional groups present in the LbL coating.

In accordance with the invention, the above-described Antimicrobial LbL coating of the Invention preferably further comprises at least one capping layer of a polyionic material, more preferably at least one capping bilayer of two oppositely charged polyionic materials or at least one capping layer of a charged polymeric material and a non-charged polymeric material that can be non-covalently bonded to the charged polymeric material, on top of the outmost antimicrobial metal -containing layer. One or more capping layers or bilayers can be served as a diffusion barrier to control the diffusion of silver or other antimicrobial metal ions out of the antimicrobial LbL coating.

In accordance with the invention, the above-described antimicrobial LbL coating of the invention preferably is further capped with a plasma coating (i.e., a medical device with an antimicrobial metal-containing LbL coating thereon is subjected to a plasma treatment to form a plasma coating on top of the antimicrobial metal-containing LbL coating.

A medical device having an antimicrobial metal-containing LbL coating thereon can be prepared by applying the Antimicrobial metal-containing LbL coating onto a preformed medical device according to any known suitable polyelectrolyte deposition techniques.

Application of an LbL coating may be accomplished in a number of ways as described in WO 99/35520 and in U.S. patent applications Publ, Nos. 2001-0045678 and 2001-0048975. It has been discovered and disclosed in WO 99/35520 that complex and time-consuming pretreatment of a core material (medical device) is not required prior to binding of a polyionic material to the core material. By simply contacting a core material of a medical device, for example, a contact lens, with one or more solutions each containing one or more polyionic materials, an LbL coating can be formed on a medical device.

Contacting of a preformed medical device with a coating solution can occur by dripping into the coating solution or by spraying it with the coating solution. One coating process embodiment involves-solely dip-coating and optionally dip-rinsing steps. Another coating process embodiment involves solely spray-coating and spray-rinsing steps, However, a number of alternatives involve various combinations of spray- and dip-coating and rinsing steps may be designed by a person having ordinary skill in the art.

For example, a solely dip-coating process involves the steps of: (a) immersing a medical device in a first coating solution of a first polyionic material; (b) optionally rinsing the medical device by immersing the medical device in a first rinsing solution; (c) immersing said medical device in a second coating solution of a second polyionic material to form a first polyelectrolyte bilayer of the first and second polyionic materials, wherein the second polyionic material has charges opposite of the charges of the first polyionic material; (d) optionally rinsing said medical device by immersing the medical device in the rinsing solution; and (e) optionally repeating steps (a) to (d) for a number of times to form additional polyelectrolyte bilayers. A thicker LbL coating can be produced by repeating steps (a) to (d) preferably for 2 to 40 times. A preferred number of bilayers is 5 to 20 bilayers. While more than 20 bilayers are possible, it has been found that delamination may occur in some LbL coatings having an excessive number of bilayers.

The immersion time for each of the coating and rinsing steps may vary depending on a number of factors. Preferably, Immersion of the core material Into the polyionic solution occurs over a period of 1 to 30 minutes, more preferably 2 to 20 minutes, and most preferably 1 to 5 minutes, Rinsing may be accomplished in one step, but a plurality of rinsing steps can be quite efficient.

Another embodiment of the coating process is a single dip-coating process as described In U.S. patent application Publ. No. 2001-0048975. Such single dip-coating process involves dipping a core material of a medical device in a solution containing a negatively charged polyionic material and a positively charged polyionic material in an amount such that the molar charge ratio of said solutions is from 3:1 to 100:1. Multiple bilayers can be formed on a medical device by using this single dip-coating process.

Another embodiment of the coating process Involves a series of spray coating techniques. For example, a solely spray-coating process generally includes the steps of: (a) spraying a medical device with a first coating solution of a first polyionic material; (b) optionally rinsing the medical device by spraying it with a rinsing solution; (c) spraying said medical device with a second coating solution of a second polyionic material to form a first polyelectrolyte bilayer of the first and second polyionic materials, wherein the second polyionic material has charges opposite of the charges of the first polyionic material; (d) optionally rinsing said medical device by spraying it with the rinsing solution; (e) optionally repeating steps (a) to (d) for a number of times. A thicker LbL coating can be produced by repeating steps (a) to (d) preferably for 2 to 40 times.

The spray coating application may be accomplished via a process selected from the group consisting of an air-assisted atomization and dispensing process, an ultrasonic-assisted atomization and dispensing process, a piezoelectric assisted atomization and dispensing process, an electro-mechanical jet printing process, a piezo-electric jet printing process, a piezo-electric with hydrostatic pressure jet printing process, and a thermal jet printing process; and a computer system capable of controlling the positioning of the dispensing head of the spraying device on the ophthalmic lens and dispensing the coating liquid. Those spraying coatIng processes are described in U.S. patent application Publ, No. 2002-0182318, By using such spraying coating processes, an asymmetrical coating can be applied to a medical device. For example, the back surface of a contact lens can be coated with a hydrophilic and/or lubricous coating material and the front surface of the contact lens can be coated with an antimicrobial metal-containing LbL coating, It is also possible to produce a coating on a contact lens, the coating having a functional pattern so as to provide simultaneously multiple benefits to a wearer.

In the above-described processes, at least one of the first and second charged material is selected from the group consisting of charged antimicrobial metal nano-particles, charged antimicrobial metal-containing nano-particles, sliver-polyelectrolyte complexes formed between silver ions and a polycationic material having amino groups, sllver-polyelectrolyte complexes formed between silver ions and a polyionic material with sulfur-containing groups, and combinations thereof.

In accordance with the present invention, polyionic material solutions can be prepared in a variety of ways. In particular, a polyionic solution of the present invention can be formed by dissolving the polyionic materlal(s) in water or any other solvent capable of dissolving the materials. When a solvent is used, any solvent that can allow the components within the solution to remain stable in water is suitable. For example, an alcohol-based solvent can be used, Suitable alcohols can include, but are not limited to, isopropyl alcohol, hexanol, ethanol, etc. It should be understood that other solvents commonly used in the art can also be suitably used in the present invention.

Whether dissolved in water or in a solvent, the concentration of a polyionic material in a solution of the present invention can generally vary depending on the particular materials being utilized, the desired coating thickness, and a number of other factors. However, it may be typical to formulate a relatively dilute aqueous solution of polyionic material. For example, a polyionic material concentration can be between 0.001 % to 0.25% by weight, between 0.005% to 0.10% by weight, or between 0.01% to 0.05% by weight.

In general, the polyionic solutions mentioned above can be prepared by any method well known in the art for preparing solutions. For example, in one embodiment, a polyanionic solution can be prepared by dissolving a suitable amount of the polyanionic material, such as polyacrylic acid having a molecular weight of about 50,000, in water such that a solution having a certain concentration is formed, In one embodiment, the resulting solution is a 0.001M PAA solution. Once dissolved, the pH of the polyanionic solution can also be adjusted by adding a basic or acidic material. In the embodiment above, for example, a suitable amount of 1 N hydrochloric acid (HCl) can be added to adjust the pH to 2,5.

However, where a coating solution containing a first polyionic material is used to form an innermost layer of a biocompatible LbL coating of the invention on the surface of a medical device, it is desirable that the concentration of the first charged polymeric material in the solution is sufficiently high enough to increase the hydrophilicity of the LbL coating. Preferably, the concentration of the charged polymeric material in a solution for forming the innermost layer of an LbL coating is at least three folder higher than the concentration of a coating material in a coating solution for forming subsequent layers of the LbL coating. More preferably, the concentration of the charged polymeric material in a solution for forming the innermost layer of an LbL coating is at least ten folder higher than the concentration of a coating material in a coating solution for forming subsequent layers of the LbL coating.

Polycationic solutions can also be formed in a manner as described above. For example, in one embodiment, poly(allylamine hydrochloride) having a molecular weight of 50,000 to 65,000 can be dissolved in water to form a 0,001M PAH solution. Thereafter, the pH can also be adjusted to 2.5 by adding a suitable amount of hydrochloric acid.

In some embodiments of the present invention, it may be desirable to use a solution containing both polyanionic and polycationic materials within a single solution, For example, a polyanionic solution can be formed as described above, and then mixed with a polycationic solution that is also formed as described above. In one embodiment, the solutions can then be mixed slowly to form the coating solution. The amount of each solution applied to the mix depends on the molar charge ratio desired. For example, if a 10:1 (polyanion:polycation) solution is desired, 1 part (by volume) of the PAH solution can be mixed into 10 parts of the PAA solution. After mixing, the solution can also be filtered if desired.

In order to alter various characteristics of the coating, such as thickness, the molecular weight of the polyionic material including polyquats can be varied. In particular, as the molecular weight Is increased, the coating thickness generally Increases. However, If the Increase in molecular weight increase is too substantial, the difficulty in handling may also increase. As such, polyionic materials used in a process of the present invention will typically have a molecular weight Mₙ of 2,000 to 150,000. In some embodiments, the molecular weight is 5,000 to 100,000, and in other embodiments, from 75,000 to 100,000.

In addition to polyionic and non-charged polymeric materials, a coating solution for forming the bilayer or part of it, can also contain additives. As used herein, an additive can generally include any chemical or material. For example, active agents, such as antimicrobials and/or antibacterial can be added to a solution forming the bilayer, particularly when used in biomedical applications. Some antimicrobial polyionic materials include polyquaternary ammonium compounds, such as those described in U.S. Patent No. 3,931, 319 to Green et al. (eg. POLYQUAD®).

Moreover, other examples of materials that can be added to a coating solution are polyionic materials useful for ophthalmic lenses, such as materials having radiation absorbing properties. Such materials can Include, for example, visibility-tinting agents, iris color modifying dyes, and ultraviolet (U\/) light tinting dyes,

Still another example of a material that can be added to a coating solution is a polyionic material that inhibits or induces cell growth, Cell growth inhibitors can be useful in devices that are exposed to human tissue for an extended time with an ultimate intention to remove (e.g. catheters or Intra Ocular Lenses (IOL's), where cell overgrowth is undesirable), while cell growth-inducing polyionic materials can be useful in permanent implant devices (e.g. artificial cornea).

When additives are applied to a coating solution, such additives, preferably, have a charge. By having a positive or negative charge, the additive can be substituted for the polyionic material In solution at the same molar ratio. For example, polyquaternary ammonium compounds typically have a positive charge. As such, these compounds can be substituted into a solution of the present invention for the polycationic component such that the additive is applied to the core material of an article in a manner similar to how a polycationic would be applied.

A preferred number of bilayers In an antimicrobial LbL coating of the invention are 5 to 20 bilayers. While more than 20 bilayers are possible, it has been found that delamination may occur in some LbL coating having excessive number of bilayers.

An antimicrobial LbL coating of the invention can be formed from at least one polyionic material, preferably two polyionic materials having charges opposite to each other.

An antimicrobial LbL coating of the invention preferably comprises at least one layer of a lubricious coating material which is selected from the group consisting of PAMAM dendrimers, PAAm-co-PAA, PVP-co-PAA, glycosaminoglycanes, fucoidan, poly-aspartic acid, poly-glutamic acid, carboxymethyl cellulose, carboxymethyl dextrans, alginates, pectins, gelian, carboxyalkyl chitins, carboxymethyl chitosans, sulfated polysaccharides, glucoproteins, and aminoalkylated polysaccharides,

Silver ions can be reduced to silver or silver nano-particles either by means of a reducing agent or by means of heating (e.g., autoclave) or by UV irradiation, During the manufacturing of medical devices, for example, contact lenses, autoclave can be used to sterilize the medical devices while reducing silver ions into silver nano-particles.

A medical device of the invention can also be made by first applying an antimicrobial metal-containing LbL coating (described above) to a mold for making a medical device and then transfer-grafting the antimicrobial metal-containing LbL coating to the medical device made from the mold, in substantial accordance with the teachings of U.S. patent application Publ. No. 2001-0045676.

Methods of forming mold sections for cast-molding a contact lens are generally well known to those of ordinary skill in the art. The process of the present invention is not limited to any particular method of forming a mold. In fact, any method of forming a mold can be used in the present invention. However, for illustrative purposes, the following discussion has been provided as one embodiment of forming a contact lens mold on which an antimicrobial metal-containing LbL coating can be formed in accordance with the present invention.

In general, a mold comprises at least two mold sections (or portions) or mold halves, i.e. first and second mold halves. The first mold half defines a first optical surface and the second mold half defines a second optical surface. The first and second mold halves are configured to receive each other such that a contact lens forming cavity is formed between the first optical surface and the second optical surface. The first and second mold halves can be formed through various techniques, such as injection molding. These half sections can later be joined together such that a contact lens-forming cavity is formed therebetween, Thereafter, a contact lens can be formed within the contact lens-forming cavity using various processing techniques, such as ultraviolet curing.

Examples of suitable processes for forming the mold halves are disclosed in U.S. Patent Nos. 4,444,711 to Schad; 4,460,534 to Boehm et al.; 5,843,346 to Morrill; and 5,894,002 to Boneberger et al.

Virtually all materials known in the art for making molds can be used to make molds for making contact lenses. For example, polymeric materials, such as polyethylene, polypropylene, and PMMA can be used. Other materials that allow UV light transmission could be used, such as quartz glass.

Once a mold is formed, a transferable antimicrobial metal-containing LbL coating of the invention (described above) can be applied onto the optical surface (inner surface) of one or both mold portions by using the above-described LbL deposition techniques. The inner surface of a mold portion is the cavity-forming surface of the mold and in direct contact with lens-forming material. A transferable antimicrobial metal-containing LbL coating can be applied onto the mold portion defining the posterior (concave) surface of a contact lens or on the mold section defining the anterior surface of a contact lens or on both mold portions.

Once a transferable antimicrobial metal-containing LbL coating is applied onto the optical surface of one or both mold portions, a lens material can then be dispensed into the contact lens forming cavity defined by the assembled mold halves. In general, a lens material can be made from any polymerizable composition. In particular, when forming a contact lens, the lens material may be an oxygen-permeable material, such as fluorine- or siloxane-containing polymer. For example, some examples of suitable substrate materials include, but are not limited to, the polymeric materials disclosed in U,S, Patent No. 5,760,100 to Nicolson et al.. The lens material can then be cured, i.e. polymerized, within the contact lens-forming cavity to form the contact lens, whereby at least a portion of the transferable coating detaches from the optical surface and reattaches to the formed contact lens.

Thermal curing or photo curing methods can be used to curing a polymerizable composition in a mold to form an ophthalmic lens. Such curing methods are well-known to a person skilled in the art.

An antimicrobial metal-containing LbL coating of the present invention may find particular use in extended-wear contact lenses. The LbL coating of the invention may have a minimal adverse effects on the desirable bulk properties of the lens, such as oxygen permeability, ion permeability, and optical properties. Moreover, the out diffusion of silver or other antimicrobial metals from the antimicrobial metal-conteining LbL coating of the present invention Is believed to be minimized. It is surprised to find that although an antimicrobial LbL coating of the invention contains silver nano-particles instead of silver lens, it still imparts to a medical device a desired level of antimicrobial activity.

The invention, in another aspect, provides a method for forming an antimicrobial metal-containing LbL coating on a medical device. The method comprises alternatively applying, in no particular order, one layer of a first charged material and one layer of a second charged material having charges opposite of the charges of the first charged material onto a medical device to form the antimicrobial LbL coating, wherein at least one of the first and second charged material is selected from the group consisting of charged antimicrobial metal nano-particles, charged antimicrobial metal-containing nanoparticules, silver-polyelectrolyte complexes formed between silver ions and a polycationic material having amino groups, silver-polyelectrolyte complexes formed between silver ions and a polyionic material having sulfur-containing groups, and combinations thereof.

The previous disclosure will enable one having ordinary skill in the art to practice the invention. In order to better enable the reader to understand specific embodiments and the advantages thereof, reference to the following examples is suggested.

### Example 1

### Contact angle

The contact angle generally measures the surface hydrophilicity of a medical device, e.g., a contact lens. In particular, a low contact angle corresponds to more hydrophilic surface. Average contact angles (Sessile Drop) of contact lenses are measured using a VCA 2500 XE contact angle measurement device from AST, Inc., located in Boston, Massachusetts.

### Antimicrobial Activity Assay

Antimicrobial activity of a contact lens with or without a silver-containing antimicrobial LbL coating of the invention is assayed against *Pseudomonas aeruginosa* GSU # 3, which is isolated from a corneal ulcer, Bacterial cells of *Pseudomnas aeruginosa* GSU # 3 stored in a lyophilized state. Bacteria are grown on an tryptic soy agar slant for 18 hours at 3.7 °C, The cells are harvested by centrifugation and washed twice with sterile, Delbeco's phosphate buffered saline. Bacterial cells are suspended in PBS and adjusted to Optical Density of 10⁸ cfu. The cell suspension is serially diluted to 10³ cfu/ml.

Lenses having a sliver-containing antimicrobial LbL coating are tested against the control lenses (i.e., without a silver-containing antimicrobial LbL coating of the invention). 200µl of from 5x10³ to 1x10⁴ cfu/ml of *P. aeruginosa* GSU #3 is placed on the surface of each lens, incubate at 25°C for 24 hours. Aspirate 50 µl out of the lens, serially dilute and plate out on agar plates to determine the microbial load of each lens, At 24 hours, colony counts are taken.

Antimicrobial activity of some contact lenses with or without silver nanoparticles and/or polyquat in the lenses of the invention is also assayed against *Staphylococcus aureus* ATCC #6638. Bacterial cells of *S.* a*ureus* #6538 is stored in a lyophilized state. Bacteria are grown on an Tryptic Soy agar slant for 18 hours at 37 °C. The cells are harvested by centrifugation and washed twice with sterile, Delbeco's phosphate buffered saline. Bacterial cells are suspended in 1/10 th strength TSB and adjusted to Optical Density of 10⁸ cfu. The cell suspension is serially diluted to 10³ cfu/ml in 1/10th strength TSB.

Lenses having a silver and/or polyquat In them are tested against the control lenses (i.e., without a silver), 200 µl of from 5x10³ to 1x10⁴ cfu/ml of *S. aureus* #6538 is placed on the surface of each lens. Incubate at 25°C for 24 hours. Aspirate 50 µl out of the lens, serially dilute and plate out on agar plates to determine the microbial load of each lens. At 24 hours, colony counts are taken.

### Ag nano-particles preparation

Unless otherwise stated, Ag nano-particles are prepared by reducing AgNO₃ using NaBH₄, or ascorbic acid or salts thereof as reducing agent and PAA as stabilizer. It should be understood that the Ag reduction reaction can be carried at various temperatures, for example, at any temperature between 0°C and elevated temperature, preferably between 0°C and the room temperature, and for a period of time from a few minutes to 24 hours or longer. PAA with different molecular weight can be used. It should be also understood that UV irradiation, heating, or hydrogen can also be used to reduce Ag+ to form Ag nano-particles.

1 mL of 0.01M AgNO₃ was mixed with 0.5 mL of 4% (by weight) PAA solution. The mixture is then keep at 0 °C using ice-water mixture. Ice cold water is used to prepare 98.5 mL of 1mM NaBH₄ solution, which is also kept in 0 °C using ice-water mixture. The mixture of AgNO₃ and PAA is then added rapidly into 98.5 mL of 1 mM NaBH₄ solution with vigorous stirring. The beaker was surrounded by ice to keep at about 0 °C.

The above described preparation procedure was used for preparing several batches of silver nanoparticles under various conditions listed in Table 1. The prepared Ag nano-partlcle solutions generally appear yellowish-gold in color and with a UV peak around 410 nm, depending on fabrication conditions.

**Table 1**

| Sample | Volume of | | | Stirring time | pH of colloids |
|---|---|---|---|---|---|
| | AgNO₃¹ | PAA² | Na₃BH₄ | | |
| A | 1mL | 0.5mL | 98.5mL | 1 h | 6.5 |
| B | 1mL | 0.5mL | 98.5mL | 2 h | 6.5 |
| C | 1mL | 0.5mL | 98.5mL | 12 h | 6.3 |
| D | 0,5mL | 0.5mL | 99mL | 12 h | 6.3 |
| E | 1mL | 0.5mL | 98.5mL | 13 h | 5.6 |
| F | 1mL | 0.5mL | 98.5mL | 12 h | 7.0 |

| | | | | | |
|---|---|---|---|---|---|
| 1. [AgNO₃] = 0.01 M; 2. [PAA] = 4% (wt/v): 3. [NaBH₄] =1 mM | | | | | |

### Example 2

*Polyacrylic acid (PAA) solution:* A solution of polyacrylic acid having a molecular weight of about 2,000, from PolyScience, is prepared by dissolving a suitable amount of the material in water to form a 4% PAA solution.

***Poly(diallyldimethylammonium chloride) (PDDA) solution:*** A solution of PDDA having a molecular weight of 400,000 to 500,00 from Aldrich, is prepared by dissolving a suitable amount of the material in water to form a 0.5% PDDA solution. The pH is adjusted by adding 0.1M NaOH solution until the pH is about 8.0.

A coating having multiple bilayers of PODA/Ag-NP is formed on a glass slide. The glass slide is dipped in the PDDA solution for 10 min. The glass slide with a first layer of PDDA is then dipped in the PAA solution for 10 minutes. Then the glass slide is dipped again in PDDA for 10 min and then dipped in the Ag-NP solution for 10 minutes. Finally, the steps of dipping In the PDDA solution for 10 minutes followed by dipping in the Ag-NP solution for 10 minutes are repeated for a desired number of times to build up a desired number of bilayers of PDDA/Ag-NP on the lens (or silicon wafer). There is rinsing step involved in the above coating process.

Bulling up of multiple bilayers of PDDA/Ag-NP can be monitored by using UV/visible absorption spectroscopy. Using silver nano-particles from Sample F, a coating with an increasing number of bilayers of PDDA/Ag-NP (from 1 to 10 bilayers)) are successfully built up on a substrate. The absorbance at 411 nm (around the absorption peak) of the coating increases linearly as the number of bilayers of PDDA/Ag-NP increases. The presence of Ag-Np in the coating was also confirmed by using AFM.

### Example 3

*Polyacrylic acid (PAA) solution:* A solution of polyacrylic acid having a molecular weight of about 90,000, from PolyScience, is prepared by dissolving a suitable amount of the material in water to form a 0.001 M PAA solution, The PAA concentration is calculated based on the repeating unit in PAA. Once dissolved, the pH of the polyanionic PAA solution is adjusted by adding 1 N hydrochloric acid until the pH is about 2.5.

*Poly(diallyldimethylammonium chlorine) (PDDA) solution:* PDDA Is a polyquat. A solution of PDDA having a molecular weight of 400,000 to 500,00 from Aldrich, is prepared by dissolving a suitable amount of the material in water to form a 0.5% PDDA solution. The pH is adjusted by adding 0.1M NaOH solution until the pH is about 8.0.

A coating having multiple bilayers of PDDA/Ag-NP is formed on a soft contact lens made of a fluorosiloxane hydrogel material, lotrafilcon A (CIBA Vision). The contact lens is dipped in the PAA solution (0.001 M, pH 2.5) for 30 minutes to form a first layer on the lens. The lens with a first layer of PAA is then dipped In the PDDA solution (0.5%, pH 8.0) for 5 minutes and then dipped in the Ag-NP solution for 5 minutes. Finally, the steps of dipping in the PDDA solution for 5 minutes followed by dipping in the Ag-NP solutions for 5 minutes are repeated for a desired number of times to build up a desired number of bilayers of PDDA/Ag-NP on the lens (or silicon wafer). There is rinsing step involved in the above coating process.

Using Ag-NP from Sample A, a coating with 10 bilayers of PDDA/Ag-NP was made. The coated lenses were autoclaved in water or in PBS. An UV peak at about 410 nm, characteristic of Ag NP, was observed for lens both autoclaved in water or in PBS. No peak at about 410 nm was observed from the packaging/storage solution (water or PBS) of theses lenses, indicating no detectable amount of Ag-NP in the packaging/storage solution (water or PBS). Or in other words, by UV method, no detectable amount of Ag-NP is leaching from the lens to packaging/storage solution (water or PBS). As listed in Table 2, the coated lenses are hydrophilic with contact angles of 50∼60 degrees, as compared to the uncoated lenses with a contact angle of about 110 degrees.

**Table 2**

| Autoclave medium | water | PBS |
|---|---|---|
| Contact angle^{*} | 81±4 | 54±5 |
| Bacterial Inhibition^{#} | 99.9% | 69.9% |

| | | |
|---|---|---|
| ^{*}: Average contact angle from 3 lenses. ^{#}: Averaged CFU/lens for control tenses is about 2.9x10⁴. | | |

Antimicrobial activity of a contact lens with a silver-containing antimicrobial LbL coating of the Invention was assayed against *Pseudomonas aeruginosa* GSU # 3 according to the procedure described in Example 1. The control lenses were Lotrafilcon A contact lenses without a silver-containing antimicrobial LbL coating. All lenses with an antimicrobial LbL coating of the invention, which are autoclaved in either water or PBS, show antimicrobial activity, characterized by at least a 3-log reduction (99.9% inhibition) of viable cells as compared to the control lenses (Table 2).

### Example 4

Using the same PAA and PDDA solution and the coating procedure as in example 3, and using Ag-NP of Sample B, a coating with 10 bilayers of PDDA/Ag-NP was made. The coated lenses were autoclaved in water or in PBS, An UV peak at about 410 nm, characteristic of Ag NP, was observed for lens both autoclaved in water or in PBS. No peak at about 410 nm was observed from the packaging/storage solution (water or PBS) of theses lenses, indicating no detectable amount of Ag-NP in the packaging/storage solution (water or PBS). Or in other words, by UV method, no detectable amount of Ag-NP is leaching from the lens to packaging/storage solution (water or PBS). As listed in Table 3, the coated lenses are hydrophilic with contact angles of about 60 degrees, as compared to the uncoated lenses with a contact angle of about 110 degrees.

**Table 3**

| Autoclave medium | water | PBS |
|---|---|---|
| Contact angle^{*} | 61±4 | 59±9 |
| Bacterial Inhibition^{#} | 99.9% | 99.9% |

| | | |
|---|---|---|
| ^{*}: Average contact angle from 3 lenses. ^{#}: Averaged CFU/lens for control lenses is about 2.9x10⁴. | | |

Antimicrobial activity of a contact lens with a sliver-containing antimicrobial LbL coating of the invention was assayed against *Pseudomonas aeruginosa* GSU # 3 according to the procedure described in Example 1, The control lenses were Lotrafilcon A contact lenses without a silver-containing antimicrobial LbL coating. All lenses with an antimicrobial LbL coating of the invention, which are autoclaved in either water or PBS, show antimicrobial activity, characterized by at least a 3-log reduction (99.9% Inhibition) of viable cells as compared to the control lenses (Table 3).

### Example 5

Using the same PAA and PDDA solution and the coating procedure as in example 3, and using Ag-NP from Sample C, a coating with 10 bilayers of PDDA/Ag-NP was made, The coated lenses were autoclaved in water or In PBS. An UV peak at about 410 nm, characteristic of Ag NP, was observed for lens both autoclaved in water or in PBS. No peak at about 410 nm was observed from the packaging/storage solution (water or PBS) of theses lenses, indicating no detectable amount of Ag-NP in the packaging/storage solution (water or PBS). Or in other words, by UV method, no detectable amount of Ag-NP is leaching from the lens to packaging/storage solution (water or PBS). As listed in Table 4, the coated lenses are hydrophilic with contact angles of about 50 degrees, as compared to the uncoated lenses with a contact angle of about 110 degrees. All lenses passed Sudan black staining test.

**Table 4**

| Autoclave Medium | water | PBS |
|---|---|---|
| Contact angle^{*} | 49±8 | 53±8 |
| Bacterial Inhibition^{#} | 99.9% | 99.9% |

| | | |
|---|---|---|
| ^{*}: average contact angle from 3 lenses. ^{#}: Averaged CFU/lens for control lenses is about 2.9x10⁴, | | |

Antimicrobial activity of a contact lens with a silver-containing antimicrobial LbL coating of the invention was assayed against *Pseudomonas aeruginosa* GSU # 3 according to the procedure described in Example 1. The control lenses were Lotrafilcon A contact lenses without a silver-containing antimicrobial LbL coating. All lenses with an antimicrobial LBL. coating of the invention, which are autoclaved in either water or PBS, show antimicrobial activity, characterized by at least a 3-log reduction (99.9% inhibition) of viable cells as compared to the control lenses (Table 4).

### Example 6

Using the same PAA and PDDA solution and the coating procedure as in example 3, and using Ag-NP from Sample E, a coating with 10 bilayers of PDDA/Ag-NP was made. The coated lenses were autoclaved in water or in PBS. An UV peak at about 410 nm, characteristic of Ag NP, was observed for lens both autoclaved in water or in PBS. No peak at about 410 nm was observed from the packaging/storage solution (water or PBS) of theses lenses, indicating no detectable amount of Ag-NP in the packaging/storage solution (water or PBS). Or in other words, by UV method, no detectable amount of Ag-NP Is leaching from the lens to packaging/storage solution (water or PBS). As listed in Table 5, the coated lenses are hydrophilic with contact angles of about 50 degrees, as compared to the uncoated lenses with a contact angle of about 110 degrees. All lenses passed Sudan black staining test.

**Table 5**

| Autoclave medium | water | PBS |
|---|---|---|
| Contact angle^{*} | 49±8 | 53±8 |
| Bacterial Inhibition^{#} | 99.9% | 99.9% |

| | | |
|---|---|---|
| ^{*}: Average contact angle from 3 lenses. ^{#}: Averaged CFU/lens for control lenses is about 2.9x10⁴. | | |

Antimicrobial activity of a contact lens with a silver-containing antimicrobial LbL coating of the invention was assayed against *Pseudomonas aeruginosa* GSU # 3 according to the procedure described in Example 1. The control lenses were Lotrafilcon A contact lenses without a silver-containing antimicrobial LbL coating. All lenses with an antimicrobial LbL coating of the invention, which are autoclaved in either water or PBS, show antimicrobial activity, characterized by at least a 3-log reduction (99.9% inhibition) of viable cells as compared to the control lenses (Table 5).

### Example 7

*Poly(ethyleneimine) (PEI) solution:* A solution of PEI having a molecular weight of about 70,000 from Polyscience, is prepared by dissolving a suitable amount of the material in water to form a 0.001 M PEI solution. The PEI concentration is based on the repeating unit in PEI. The pH of the PEI solution is adjusted by adding 0.1M nitric acid until the pH is about 8.0.

*Polyacrylic acid-silver (PAA-Ag) solution:* A PAA-Ag solution is prepared by dissolving a suitable amount of PAA (molecular weight of 90,000, from PolyScience) and silver nitrate (AgNO₃) in water to form a 0.01 M of PAA and 0.01 M of AgNO₃. The PAA concentration is calculated based on the repeating unit in PAA. Once dissolved, the pH of the PAA-Ag solution is adjusted by adding 1 N nitric acid until the pH is about 2.5.

*Sodium borohydride (NaBH₄) solution:* a solution of NaBH₄ solution is prepared by dissolving a suitable amount of sodium borohydride solid (from Aldrich) in water to form 0.001 M NaBH₄ solution.

A coating having multiple bilayers of PAA-Ag/PEI is formed on a silicone wafer and a soft contact lens made of a fluorosiloxane hydrogel material, lotrafilcon A (CIBA Vision). The contact lens (and also the silicone wafer) is dipped in four PAA solutions (0.001M, pH 2.5) for 5 min each and a total of 20 minutes to form a first layer on the lens. The lens with a first layer of PAA is then dipped in the PAA-Ag solution for 5 minutes and then dipped in the PEI solution for 5 minutes. Then the steps of dipping in the PAA-Ag solution for 5 minutes followed by dipping in the PEI solution for 5 minutes are repeated for a desired number of times to build up a desired number of bilayers of PAA-Ag/PEI on the lens (or silicon wafer). Finally, the lens is dipped In NaBH₄ solution for 5 min. There is rinsing step Involved in the above coating process. All the lenses are then released and autoclaved in water or in PBS.

The coating thickness on silicone wafer is about 21 nm as measured by ellipsometry. As listed in Table 6, the coated lenses are hydrophilic with contact angles of 30∼65 degrees, as compared to the uncoated lenses with a contact angle of about 110 degrees. All lenses passed Sudan black staining test.

**Table 6**

| Autoclave medium | water | PBS |
|---|---|---|
| Contact angle^{*} | 29 | 65 |
| Bacterial inhibition^{#} | 99.9% | 97.5% |

| | | |
|---|---|---|
| ^{*}: Average contact angle from 3 lenses ^{#}: Averaged CFU/lens for control lenses is about 1.0x10⁴. | | |

Antimicrobial activity of a contact lens with a silver-containing antimicrobial LbL coating of the invention was assayed against *Pseudomonas aeruginosa* GSU # 3 according to the procedure described in Example 1. The control lenses were Lotrafilcon A contact lenses without a silver-containing antimicrobial LbL coating. All lenses with an antimicrobial LbL coating of the invention, which are autoclaved in either water or PBS, show antimicrobial activity, characterized by a 97.5% to 99.9% inhibition of viable cells as compared to the control lenses (Table 6).

### Example 8

*Polyacrylic acid (PAA) solution:* A solution of polyacrylic acid having a molecular weight of about 90,000, from PolyScience, is prepared by dissolving a suitable amount of the material In water to form a 0.001 M PAA solution. The PAA concentration is calculated based on the repeating unit in PAA. Once dissolved, the pH of the polyanionic PAA solution is adjusted by adding 1 N nitric acid until the pH is about 2.5.

*Poly(ethyleneimine)-Ag⁺ (PEI-Ag⁺) solution:* A PEI-Ag⁺ solution is prepared by dissolving a suitable amount of PEI (molecular weight of 70,000, from PolyScience) and silver nitrate (AgNO₃) in water to form a 0.01 M of PEI and 0.001 M of AgNO₃. The PEI concentration is calculated based on the repeating unit in PEI. Once dissolved, the pH of the PEI-Ag⁺ solution is adjusted by adding 1N nitric acid until the pH is about 6.0.

*Polyacrylic acid-silver (PAA-Ag) solution:* The same as in Example 7

*Sodium borohydride (NaBH₄) solution:* The same as in Example 7

A coating having multiple bilayers of PAA-Ag/PEI-Ag* is formed on a silicone wafer and a soft contact lens made of a fluorosiloxane hydrogel material, lotrafilcon A (CIBA Vision). The contact lens (and also the silicone wafer) Is dipped in four PAA solutions (0.001 M, pH 2.5) for 5 min each and a total of 20 minutes to form a first layer on the lens. The lens with a first layer of PAA is then dipped in the PAA-Ag solution for 6 minutes and then dipped in the PEI-Ag+ solution for 5 minutes. Then the steps of dipping in the PAA-Ag solution for 5 minutes followed by dipping in the PEI-Ag⁺ solution for 5 minutes are repeated for a desired number of times to build up a desired number of bilayers of PAA-Ag/PEI-Ag⁺ on the lens (or silicon wafer). Finally, the lens is dipped in NaBH₄ solution for 5 min. There is rinsing step involved in the above coating process. All the lenses are then released and autoclaved in water or in PBS.

The coating thickness on silicone wafer is about 24 nm as measured by ellipsometry. As listed In Table 7, the coated lenses are hydrophilic with contact angles of about 20 degrees, as compared to the uncoated lenses with a contact angle of about 110 degrees. All lenses passed Sudan black staining test.

**Table 7**

| Autoclave medium | water | PBS |
|---|---|---|
| Contact angle^{*} | 18 | 23 |
| Bacterial inhibition^{#} | 99.9% | 99.9% |

| | | |
|---|---|---|
| ^{*}: Average contact angle from 3 lenses ^{#}: Averaged CFU/lens for control lenses is about 1,0x10⁴. | | |

Antimicrobial activity of a contact lens with a silver-containing antimicrobial LbL coating of the invention was assayed against *Pseudomonas aeruginosa* GSU # 3 according to the procedure described in Example 1. The control lenses were Lotrafilcon A contact lenses without a silver-containing antimicrobial LbL coating. All lenses with an antimicrobial LbL coating of the invention, which are autoclaved In either water or PBS, show antimicrobial activity, characterized by at least a 3-log reduction (99.9% inhibition) of viable cells as compared to the control lenses (Table 7).

### Example 9

*Polyacrylic acid (PAA) solution:* The same as in Example 7.

*Poly(ethyleneimine)-Ag⁺(PEI-Ag⁺) solution:* A PEI-Ag+ solution is prepared by dissolving a suitable amount of PEI (molecular weight of 70,000, from PolyScience) and silver nitrate (AgNO₃) in water to have a PEI concentration of about 7 mM and a AgNO₃ concentration of about 3 mM (solution A) or to have a PEI concentration of about 1 mM and a AgNO₃ concentration of about 1 mM (solution B). The PEI concentration is calculated based on the repeating unit In PEI, Once dissolved, the pH of the PEI-Ag⁺ solution is adjusted by adding 1N nitric acid until the pH is about 6.0.

*Sodium borohydride (NaBH₄) solution:* The same as in Example 7.

A coating having multiple bilayers of PAA/PEI-Ag^{*} is formed on a silicone wafer and a soft contact lens made of a fluorosiloxane hydrogel material, lotrafilcon A (CIBA Vision). The contact lens (and also the silicone wafer) is dipped in four PAA solutions (0.001M, pH 2.5) for 5 min each and a total of 20 minutes to form a first layer on the lens. The lens with a first layer of PAA is then dipped In the PAA solution for 5 minutes and then dipped in the PEI-Ag⁺ solution for 5 minutes. Then the steps of dipping in the PAA solution for 5 minutes followed by dipping In the PEI-Ag⁺ solution for 5 minutes are repeated for a desired number of times to build up a desired number of bilayers of PAA/PEI-Ag⁺ on the lens (or silicon wafer). Half of the lenses are then released and autoclaved in water or in PBS. Finally, the rest half of the lenses are dipped in NaBH₄ solution for 5 min and the lenses are then released and autoclaved in water or in PBS. There is rinsing step involved in the above coating process. As listed in Tables 8 and 9, the coated lenses are hydrophilic with contact angles of 20∼65 degrees, as compared to the uncoated lenses with a contact angle of about 110 degrees. All lenses passed Sudan black staining test.

**Table 8**

| Autoclave medium | water | PBS | water | PBS |
|---|---|---|---|---|
| Reduction by NaBH₄ before autoclave | No | No | Yes | Yes |
| Contact angle^{*} | 30 | 45 | 60 | 65 |
| Bacterial Inhibition^{#} | 99.9% | 98% | 99.9% | 0% |

| | | | | |
|---|---|---|---|---|
| ^{*}: Average contact angle from 3 lenses ^{#}: Averaged CFU/lens for control lenses is about 1.0x10⁴. | | | | |

**Table 9**

| Autoclave medium | water | PBS | water | PBS |
|---|---|---|---|---|
| Reduction by NaBH₄ before autoclave | no | no | yes | yes |
| Contact angle^{*} | 30 | 18 | 29 | 22 |
| Bacterial Inhibition^{#} | 0% | 0% | 0% | 0% |

| | | | | |
|---|---|---|---|---|
| ^{*}: Average contact angle from 3 lenses ^{#}: Averaged CFU/lens for control lenses is about 1.0×10⁴, | | | | |

Antimicrobial activity of a contact lens with a silver-contalning antimicrobial LbL coating of the invention was assayed against *Pseudomonas aeruginosa* GSU # 3 according to the procedure described in Example 1, The control lenses were Lotrafilcon A contact lenses without a silver-containing antimicrobial LbL coating. Depending on the silver concentration and/or PEI/Ag⁺ concentration ratio, the lenses in this example may or may not show antimicrobial activity based on in-vitro test. Lack of antimicrobial activity for some lenses is presumably due to low Ag⁺ concentration in the coating,

### Example 10

### Antimicrobial coating with a synergistic functions of polyquat and silver nano particles

Two types of coatings are applied onto lotrafilcon A silicon hydrogel contact lenses:
(1) A coating with both polyquat (PDDA) and PAA-stabilized silve nano-particles (PAA-AgNP), The concentration of PDDA can range from 0.1 mM to 100mM, preferably from 1mM to 30 mM. The concentration of PAA-AgNP can range from 0.01 mM to 10 mM, preferably from 0.1 mM to 5 mM. As an example, a coating with 10 bilayers of PDDA/PAA-AgNP is made and autoclaved in PBS. 0.1 mM of PAA-AgNP solution is prepared according to the procedure described Example 1.1 mM PAA solution and 0.5% PDDA solution are prepared according to the procedure described in Example 3. Other concentrations of PAA-AgNP and PDDA solutions can also be used
(2) A coating with only polyquat (prepared from PDDA and PAA), but no silver nanoparticles. As an example, a coating with 10 bilayers of PDDA/PAA is made and autoclaved in PBS. 0.1 mM PAA solution and 0.5% PDDA solution are prepared according to the procedures described in Example 3. Other concentrations of PAA-AgNP and PDDA solutions can also be used.

Antimicrobial activity of contact lenses with both coating (1) and (2) are assayed against *Pseudomonas aeruginosa* GSU # 3 and *Staphylococcus aureus* ATCC #6538 according to the procedure described in Example 1. Lenses with both coatings show high antimicrobial activity against *Staphylococcus aureus,* characterized by 100% inhibition of viable cells. However, lenses with coating (2) (with PDAA but no silver) show much lower Inhibition against *Pseudomonas aeruginosa* as compared to lenses with coating (1) (with both PDDA and silver). This data indicates the potential advantage of having both PDDA and silver in the coating.

### Example 11

### Coating with or without plasma coating on top of PDDA/PAA-AgNP.

A coating with 5 bilayers of PDDA/PAA-AgNP on Lotrafilcon A contact lenses is prepared, with or without a Plasma coating layer on top of the 5 bilayers of PDDA/PAA-AgNP. The coating process also includes a primer dip coating step In PAA solution. After coating, some of lenses are released into PBS and then autoclaved in PBS. Some of the lenses are released into water, then dried and plasma coated. In this example, the concentration of PDDA used is 0.1 wt% and the concentration of PAA-AgNP is 0.1 mM.

The average silver concentration In the coated lenses is about 52 ppm, as determined by instrumental neutron activation analysis (INAA). Antimicrobial activity of the both lenses with or without plasma coating on top of the 5 bilayers of PDDAIPAA-AgNP are assayed against *Staphylococcus aureus* ATCC #6538 according to the procedure described In Example 1. Both lenses show 100% inhibition of viable cells.

The lenses with plasma coating on top of the PDDA/PAA-AgNP is tried on-eyes in an over night trial modality. After the lenses are worn, the activity of worn lenses is assay by challenging the worn lenses with *Staphylococcus aureus (Saur31)* or *Pseudomonas aeruginosa* (*Paer6294*). The worn lenses maintain efficacy against both Pseudomonas and *Staphylococcus,* giving 66% and 57% reduction of viable adhesion for Pseudomonas and *Staphylococcus,* respectively.

### Example 12

### A coating with PDDA/PAA-AgNP on top of plasma coating

A coating with 5 bilayers of PDDA/PAA-AgNP on is prepared on plasma coated Lotrafilcon A contact lenses, After coating, the lenses are released into PBS and then autoclaved In PBS. As the same as In example B, the concentration of PDDA used is 0.1 wt% and the concentration of PAA-AgNP is 0.1 mM.

The average silver concentration in the coated lenses is about 7 ppm, as determined by Instrumental neutron activation analysis (INAA). Antimicrobial activity of the coated lenses (with 5 bilayers of PDDA/PAA-AgNP on top of plasma coating) are assayed against *Staphylococcus aureus* ATCC #6538 according to the procedure described In Example 1 and show 140% Inhibition of viable cells.

### Example 13

### A non water rinse dip coating process

A coating with polyquat and silver nano particles on a Lotrefilcon A contact lens is prepared by alternatively dipping the lens in PDDA-containing solution and in PAA-AgNP containing solution, without water rinse steps between dips in PDDA-containing solutions and in PAA-AgNP containing solution.

The common practice in LbL dip coating normally involves water rinse step or steps in between dips in polycationic solution and polyanionic solution. It is desirable to reduce or to eliminate the number of water rinse steps for a more efficient coating process. An improved coating process is discovered by using PDDA dominated solution (instead of PDDA solution).

PDDA dominated solution (referred to as PDDAd) is prepared by mixing appropriate volume of PAA-AgNP solution into PDDA solution. The ratio of PDDA to PAA-AgNP can be controlled from 1/1 to 1/0.001. preferably from 1/0.1 to 1/0.01. It is also discovered that of order of mixing may be important. Mixing of PAA-AgNP solution into PDDA solution is preferred. As an example, a PDDAd solution is made by mixing equal volume of 0.2 mM of PAA-AgNP solution into 2 mM PDDA solution. 0.2 mM of PAA-AgNP solution is prepared according to the procedure described Example 1. 2 mM of PDDA solution is prepared according to the procedure described in Example 3,

Four kinds of coatings with different numbers of bialyers of PDDA/PAA-AgNP are applied onto Lotrafilcon A contact lenses. The numbers of bilayers are 2, 3, 4, and 5, respectively. Lenses are autoclaved in PBS.

Antimicrobial activity of contact lenses with different numbers of bilayers are assayed against *Staphylococcus aureus* ATCC #6536 according to the procedure described in Example 1. All lenses show antimicrobial activity against *Staphylococcus aureus* (as shown in Table 10)

**Table 10**

| Number of bilayers of PDDAIPAA-AgNP | 2 | 3 | 4 | 5 |
|---|---|---|---|---|
| Bacterial Inhibition against *Staphylococcus aureus^{*}* | 99.8% | 100% | 99.8% | 99.9% |

| | | | | |
|---|---|---|---|---|
| ^{*}: Average contact angle from 3 lenses. | | | | |

### Example 14

### A visitinted lens with PDDA/PAA-AgNP coating

Depending on coating conditions, lenses with PDDAIPAA-AgNP coating may appear to be clear or having a yellowish color. To prepare visitinted lenses, uncoated green visitinted or blue visitinted lenses are also used in the coating under coating conditions similar to that described in Example 13. Green visitinted or blue visitinted lenses are achieved in this manner.

Uncoated green visitinted or blue visitinted Lotrafilcon A lenses are fabricated from Lotrafilcon A formulations containing green (copper phthalcyanine green) or blue (copper phthalcyanine blue) pigments.

### Example 15

### A coating consisting of PEI-AgNP and with ascorbic acid as reducing agent to form silver nano-particles (AgNP)

A coating with 2 bilayers of PEI-AgNP /PAA-AgNP on Lotrafilcon A contact lenses is prepared, with or without a plasma coating layer on top of the 5 bilayers of PEI-AgNP/PAA-AgNP.

In search for other reducing agent (other than sodium borohydride, NaBH₄).it is discovered that ascorbic acid (or vitamin C) can reduce silver ions into silver particles. It is also discovered that silver particles can form when mixing silver nitrate into poly(ethylene imine) (PEI) solution, However, the color of the PEI-AgNP solution deepen as time go on and the UV absorption intensity of silver nano-particles peaks around 400 nm also increase with time. It is then further discovered that ascorbic acid (or vitamin C, VC) could speed up and/or stabilize the process of silver nano-particle formation in PEI-AgNO₃ system. The solution of PEI-AgNP-VC is stable at least over the course of 2 days and the UV adsorption intensity remains also fairy consistent over the course of at least 2 days.

In this example, the lenses are dipped first in a PAA-Ag solution, followed by a dip in PEI-AgNP-VC solution, then in PAA-Ag solution again. After certain number of bilayers of PEI-AgNP-VC/PAA-Ag (for example, 2 bilayers in one example), the lenses are dipped one time in a VC solution. Water rinse steps are used between dips. After coating, lenses are released into water, then dried and plasma coated.

The concentration silver concentration of PEI-AgNP-VC can range from 0.01 mm to 10mM, preferably from 0.1 mM to 2 mM. The concentration of PAA-Ag can range from 0.01 mM to 100mM, preferably from 0.1mM to 10mM. In this example, the silver concentration of PEI-AgNP-Vc is 0.2 mM and the concentration of PAA-Ag is 1 mM.

The average silver concentration in the coated lenses is about 57 ppm, as determined by atomic adsorption (AA). Antimicrobial activity of the lenses are assayed against *Staphylococcus aureus* ATCC #6538 according to the procedure described in Example 1. The lenses show 93∼95% inhibition of viable cells.

The lenses are also subjected to 30 PBS rinsing cycles (one rinse per day in PBS) and then assayed again against *Staphylococcus aureus* ATCC #6538. After 30 cycles, the lenses maintain their activity with about ∼95% inhibition of viable cells.

### Example 18

### Control the color of silver nanopartic/es solutions

Normally, yellow is the color of a silver nano-particles solution formed in aqueous solution using reducing agent (e.g. NaBH₄), It is unexpected discovered that colors other than yellow can be generated by exposing a PAA-AgNO₃ mixture solution to a certain UV treatment.
1. Aqua blue silver nano-particle solution:
   A solution of PAA-AgNO₃ mixture with 1:1 1 molar ratio of -COOH and AgNO₃ is prepared by dissolve calculated amount of PAA and AgNO₃ into appropriate volume of water. The pH of the solution is 3.3∼3.4 for a 10 mM solution. The solution is clear with no color, Then the solution is exposed to a LQ-400 Grobel lamp whose UV spectrum covers from 250 nm to 660 nm. The exposure time varies from 10 sec to 180 sec. It is discovered that at 35 sec exposure, the solution remains clear; after 50 sec exposure, the solution turns into aqua blue; after 180 sec exposure, the solution remains aqua blue.
   The blue color cannot be produced when the PAA-AgNO₃ mixture solution is exposed to a fluorescent tube with a UV spectrum of 350 to 440 nm.
   It is also discovered that the blue color disappear when the pH of the solution is adjusted to 2.5 using nitric acid.
2. Pink silver nano-particle solution
   Another unexpected and interesting discovery is that when the pH of the solution is first adjusted to 5.0, the solution turns from clear to pink when exposed to a LQ-400 Grobel lamp for 30 sec or longer. In addition the color progresses from light pink to medium pink and then to dark pink when the exposure time is increased from 30 seconds, to 65 seconds and then to 120 seconds.
3. Green silver nano-particle solution
   When adding a drop of 1mM NaBH₄ solution to 10mM of PAA-AgNO₃ (1;1) mixture solution, the solution turns from clear to light yellow. Interestingly, the solution then turns into green color after exposed for 65 seconds to a LQ-400 Grobel lamp.

## Claims

1. A medical device comprising a core material and an antimicrobial metal-containing layer-by-layer coating, wherein the antimicrobial metal-containing layer-by-layer coating comprises a member selected from the group consisting of: (a) one layer of charged antimicrobial metal nano-particles; (b) one layer of charged antimicrobial metal-containing nano-particles; (c) silver-polyelectrolyte complexes formed between silver ions and a polycationic material having amino groups; (d) silver-polyelectrolyte complexes formed between silver ions and a polyionic material having sulfur-containing groups; (e) silver nano-particles; and (f) combinations thereof, wherein the antimicrobial metal-containlng layer-by-layer coating is not covalently attached to the core material and Imparts to the medical device a hydrophilicity **characterized by** having an averaged contact angle of less than 80 degree.

2. A medical device of claim 1, wherein the medical device further comprises one or more antimicrobial agents selected from the group consisting of polyquats which exhibit antimicrobial activity, furanones, antimicrobial peptides, isoxazolinones, and organic selenium compounds.

3. A medical device of claim 2, wherein said one or more antimicrobial agents are covalently attached to the surface of the core material,

4. A medical device of claim 2, wherein said one or more antimicrobial agents are covalently attached to the antimicrobial metal-containing layer-by-layer coating through the reactive sites of the antimicrobial metal-containing layer-by-layer coating.

5. A medical device of claim 1, wherein the antimicrobial metal-containing layer-by-layer coating comprises one capping layer of a polyionic material

6. A medical device of claim 1, wherein the antimicrobial metal-containing layer-by-layer coating comprises one capping electrolyte bilayer of a positively charged polyionic material and a negatively charged polyionic materiel or one capping layer of a charged polymeric material and a non-charged polymeric material that can be non-covalently bonded to the charged polymeric material.

7. A medical device of any one of claims 1 to 6, wherein the antimicrobial layer-by-layer coating comprises at least one layer of an antimicrobial polyquat.

8. A medical device of any one of claims 1 to 7, wherein the medical device is a hard or soft contact lens.

9. A contact lens of claim 8, wherein the antimicrobial metal-containing layer-by-layer coating comprises: at least one layer of charged antimicrobial metal nano-particles and/or charged antimicrobial metal-containing nanoparticles; and at least one layer of a polyionic material having charges opposite of the charges of the charged antimicrobial metal nano-particles and/or charged antimicrobial metal-containing nanoparticles.

10. A contact lens of claim 9, wherein said charged antimicrobial metal nanoparticles are charged silver nanoparticles, and wherein the charged antimicrobial metal-containing nanoparticles are charged silver-containing nanoparticles.

11. A contact lens of claim 8, wherein the antimicrobial metal-containing layer-by-layer coating comprises at least one layer of a negatively-charged polyionic material and at least one layer of silver-polyelectrolyte complexes formed between silver ions and a polycationic material having amino groups.

12. A contact lens of claim 8, wherein the antimicrobial metal-containing layer-by-layer coating comprises at least one layer of a negatively-charged polyionic material and at least one layer of silver-polyelectrolyte complexes formed between silver cations and a polyionic material having sulfur-containing groups.

13. A contact lens of claim 8, wherein the antimicrobial metal-containing layer-by-layer coating comprises at least one layer of a silver-polyelectrolyte complex formed between silver ions and a polyionic material having sulfur-containing groups.

14. A contact lens of claim 8, wherein the antimicrobial metal-containing layer-by-layer coating comprises silver nano-particles.

15. A contact lens of claim 14, wherein the silver nano-particles are obtained by first forming a transitional layer-by-layer coating composed of at least one layer of a first polyionic material and at least one layer of a second polyionic material having charges opposite of the charges of the first polyionic material, wherein at least one of the first and second polyionic material is a sliver-polyelectrolyte complex formed between silver cations and a positively-charged amino group containing polyionic material, a silver-polyelectrolyte complex formed between silver cations and a polyionic material with sulfur-containing groups; and then by reducing silver ions in the transitional layer-by-layer coating by means of a reducing agent, UV irradiation or heating.

16. A contact lens of claim 8, wherein the contact lens further comprises a plasma coating on top of the antimicrobial metal-containing layer-by-layer coating.

17. A medical device of any one of claims 1 to 8, wherein the medical device is a case or container for storing an ophthalmic device or an ophthalmic solution.

18. A method for preparing a medical device having an antimicrobial metal-containing layer-by-layer coating thereon, comprising the steps of
dipping said medical device in a solution containing a first charged material and a second charged material for a desired period of time so as to obtain the antimicrobial layer-by-layer coating **characterized by** having an average contact angle of 80 degrees or less, wherein the second charged material has charges opposite of the charges of the first charged material, wherein the first charged material and the second charged material are present in an amount such that the ratio of the charges of the first charged material to the second charged material is from 3:1 to 100:1, wherein at least one of the first and second charged material is selected from the group consisting of charged antimicrobial metal nanoparticles, charged antimicrobial metal-containing nano-particles, silver-polyelectrolyte complexes formed between silver ions and a polycationic material having amino groups, silver-polyelectrolyte complexes formed between silver ions and a polyionic material having sulfur-containing groups, and combinations thereof.

19. A method of claim 18, wherein at least one of the first and second charged material is selected from the group consisting of silver-polyelectrolyte complexes formed between silver ions and a polycationic material having amino groups, silver-polyelectrolyte complexes formed between silver ions and a polyionic material having sulfur-containing groups, and combinations thereof, and wherein the method further comprises a step of reducing the silver ions in the antimicrobial layer-by-layer coating to form silver nano-particles.

20. A method of claim 19, wherein said reducing occurs by means of a reducing agent, UV irradiation, or heating.

21. A method of claim 19, comprising, prior the step of dipping, the step of completely or partially coating the surface of the medical device with at least one antimicrobial agent selected from the group consisting of a polyquat which exhibits antimicrobial activity, furanones, antimicrobial peptides, isoxazolinones, and organic selenium compounds, wherein said at least one antimicrobial agent is covalently attached to the surface of the medical device.

22. A method of claim 18, further comprising the step of covalently attaching at least one antimicrobial agent to the antimicrobial metal-containing layer-by-layer coating through the reactive sites of the antimicrobial metal-containing layer-by-layer coating, wherein said at least one antimicrobial agent is selected from the group consisting of a polyquat which exhibits antimicrobial activity, furanones, antimicrobial peptides, isoxazolinones, and organic selenium compounds.

23. A method of claim 18, further comprising the step of subjecting the medical device with the antimicrobial metal-containing layer-by-layer coating to a plasma treatment to form a plasma coating on top of the antimicrobial metal-containing layer-by-layer coating.

24. A method for producing a medical device having an antimicrobial metal-containing layer-by-layer coating thereon, wherein the medical device is a contact lens, comprising the steps of:
(a) forming a mold for making the contact lens, wherein the mold comprises a first mold portion defining a first optical surface and a second mold portion defining a second optical surface, wherein said first mold portion and said second mold portion are configured to receive each other such that a contact lens-forming cavity is formed between said first optical surface and said second optical surface;
(b) applying a transferable antimicrobial layer-by-layer coating, using a layer-by-layer polyelectrolyte deposition technique, onto at least one of said optical surface, wherein the transferable antimicrobial layer-by-layer coating comprises at least one layer of a first charged material and at least one layer of a second charged material having charges opposite of the charges of the first charged material wherein at least one of the first and second charged material is selected from the group consisting of charged antimicrobial metal nano-particles, charged antimicrobial metal-containing nanoparticles, silver-polyelectrolyte complexes formed between silver ions and a polycationic material having amino groups, silver-polyelectrolyte complexes formed between silver ions and a polyionic material having sulfur-containing groups, and combinations thereof;
(c) positioning said first mold portion and said second mold portion such that said mold portions receive each other and said optical surfaces define said contact lens forming cavity;
(d) dispensing a polymerizable composition into said contact lens-forming cavity; and
(e) curing said polymerizable composition within said contact lens-forming cavity such that the contact lens is formed, whereby said transferable antimicrobial layer-by-layer coating detaches from said at least one optical surface of said mold portion and reattaches to said formed contact lens such that said contact lens becomes coated with the antimicrobial metal-containing layer-by-layer coating having an averaged contact angle of less than 80 degrees.

25. A method of claim 24, wherein the method further comprises a step of reducing silver ions in the transferable antimicrobial layer-by-layer coating or in the silver-containing antimicrobial layer-by-layer coating to form silver nano-particles.

26. A method of claim 25, wherein said reducing occurs by means of a reducing agent, UV Irradiation, or heating.

27. A method of claim 24, further comprising the step of covalently attaching at least one antimicrobial agent to the antimicrobial layer-by-layer coating through the reactive sites of the antimicrobial layer-by-layer coating on the contact lens, wherein said at least one antimicrobial agent is selected from the group consisting of a polyquat which exhibits antimicrobial activity, furanones, antimicrobial peptides, isoxazolinones, and organic selenium compounds.

## Patentansprüche

1. Medizinalprodukt umfassend ein Kernmaterial und eine antimikrobielle Metall-enthaltende Schicht-für-Schicht Beschichtung, wobei die antimikrobielle Metall-enthaltende Schicht-für-Schicht Beschichtung ein Mitglied der Gruppe umfasst die ausgewählt sind aus: (a) einer Schicht geladener antimikrobieller Metall-Nanopartikel; (b) eine Schicht geladener antimikrobieller Metall-enthaltender Nanopartikel; (c) Silber-Polyelektrolyt-Komplexe die aus Silberionen und einem polykationischen Material mit Aminogruppen gebildet sind; (d) Silber-Polyelektrolyt-Komplexe die aus Silberionen und einem polyionischen Material mit Schwefel-enthaltende Gruppen gebildet sind; (e) Silber-Nanopartikel; und (f) Kombinationen davon, wobei die antimikrobielle Metall-enthaltende Schicht-für-Schicht Beschichtung nicht-kovalent mit dem Kernmaterial verbunden ist und dem Medizinalprodukt eine Hydrophilität verleiht, die durch einen mittleren Kontaktwinkel von weniger als 80 Grad **gekennzeichnet** ist.

2. Medizinalprodukt gemäss Anspruch 1, wobei das Medizinalprodukt ferner ein oder mehrere antimikrobielle Mittel umfasst, die aus der Gruppe bestehend aus Polyquats welche antimikrobielle Aktivität zeigen, Furanonen, antimikrobiellen Peptiden, Isoxazolinonen, und organische Selen-Verbindungen ausgewählt sind.

3. Medizinalprodukt gemäss Anspruch 2, wobei die ein oder mehreren antimikrobiellen Mittel an die Oberfläche des Kernmaterials kovalent gebunden sind.

4. Medizinalprodukt gemäss Anspruch 2, wobei die ein oder mehreren antimikrobiellen Mittel an die antimikrobielle Metall-enthaltende Schicht-für-Schicht Beschichtung durch reaktive Gruppen der antimikrobiellen Metall-enthaltenden Schicht-für-Schicht Beschichtung kovalent gebunden sind.

5. Medizinalprodukt gemäss Anspruch 1, wobei wobei die antimikrobielle Metall-enthaltende Schicht-für-Schicht Beschichtung eine Abschluss-Schicht aus einem polyionischen Material umfasst.

6. Medizinalprodukt gemäss Anspruch 1, wobei die antimikrobielle Metall-enthaltende Schicht-für-Schicht Beschichtung eine abschliessende Elektrolyt-Doppelschicht eines positive geladenen polyionischen Materials und eines negativ geladenen polyionischen Materials oder eine Abschluss-Schicht aus einem geladenen polyionischen Material und einem ungeladenen polyionischen Material umfasst das nicht-kovalent an das geladene Polymer-Material gebunden werden kann.

7. Medizinalprodukt gemäss einem der Ansprüche 1 bis 6, wobei die antimikrobielle Schicht-für-Schicht Beschichtung mindestens eine Schicht eines antimikrobiellen Polyquat umfasst.

8. Medizinalprodukt gemäss einem der Ansprüche 1 bis 7, wobei das Medizinalprodukt eine harte oder weiche Kontaktlinse ist.

9. Kontaktlinse gemäss Anspruch 8, wobei die antimikrobielle Metall-enthaltende Schicht-für-Schicht Beschichtung umfasst: mindestens eine Schicht geladener antimikrobieller Metall-Nanopartikel und/oder geladener antimikrobieller Metall-enthaltender Nanopartikel; und mindestens eine Schicht eines polyionischen Materials mit entgegengesetzten Ladungen zu den Ladungen der geladenen antimikrobiellen Metall-Nanopartikel und/oder geladenen antimikrobiellen Metall-enthaltenden Nanopartikel.

10. Kontaktlinse gemäss Anspruch 9, wobei die geladenen antimikrobiellen Metall-Nanopartikel geladene Silber-Nanopartikel sind, und wobei die geladenen antimikrobiellen Metall-enthaltenden Nanopartikel geladene Silber-enthaltende Nanopartikel sind.

11. Kontaktlinse gemäss Anspruch 8, wobei die antimikrobielle Metall-enthaltende Schicht-für-Schicht Beschichtung mindestens eine Schicht eines negativ geladenen polyionischen Materials und mindestens eine Schicht eines Silber-Polyelektrolyt-Komplexes der aus Silberionen und polykationischem Material mit Aminogruppen gebildet ist umfasst.

12. Kontaktlinse gemäss Anspruch 8, wobei die antimikrobielle Metall-enthaltende Schicht-für-Schicht Beschichtung mindestens eine Schicht eines negativ geladenen polyionischen Materials und mindestens eine Schicht eines Silber-Polyelektrolyt-Komplexes der aus Silberionen und polykationischem Material mit Schwefel-enthaltende Gruppen gebildet ist, umfasst.

13. Kontaktlinse gemäss Anspruch 8, wobei the antimikrobiellen Metall-enthaltenden Schicht-für-Schicht Beschichtung comprises at least one layer of a Silber-Polyelektrolyt-Komplex formed between Silberionen and a polyionisches Material having Schwefel-enthaltende Gruppen.

14. Kontaktlinse gemäss Anspruch 8, wobei die antimikrobielle Metall-enthaltende Schicht-für-Schicht Beschichtung Silber-Nanopartikel umfasst.

15. Kontaktlinse gemäss Anspruch 14, wobei die Silber-Nanopartikel erhalten warden in dem zuerst eine Übergangs-Beschichtung bestehend aus mindestens einer Schicht eines ersten polyionischen Materials und mindestens einer Schicht eines zweiten polyionischen Material mit entgegengesetzten Ladungen zu den Ladungen des ersten polyionischen Material umfasst, wobei mindestens eines des ersten und zweiten polyionischen Materials ein Silber-Polyelektrolyt-Komplex ist der aus Silberkationen und einem positiv-geladenen Amino-gruppen enthaltenden polyionischen Material, ein Silber-Polyelektrolyt-Komplex ist der aus Silberkationen und einem positiv-geladenen Schwefel-gruppen enthaltenden polyionischen Material; und dann durch Reduktion der Silberionen in der Übergangs Beschichtung durch ein Reduktionsmittel, UV Strahlung oder Erwärmen erfolgt.

16. Kontaktlinse gemäss Anspruch 8, wobei die Kontaktlinse ferner eine Plasma-Beschichtung über der antimikrobiellen Metall-enthaltenden Schicht-für-Schicht Beschichtung umfasst.

17. Medizinalprodukt gemäss einem der Ansprüche 1 bis 6, wobei das Medizinalprodukt eine Hülle oder ein Behälter zur Aufbewahrung eines ophthalmischen Produkts oder einer ophthalmischen Lösung ist.

18. Verfahren zum Herrichten eines Medizinalprodukts mit einer antimikrobiellen Metall-enthaltenden Schicht-für-Schicht Beschichtung, umfassend die Schritte Eintauchen des Medizinalprodukts in eine Lösung enthalten ein erstes geladenes Material und ein zweites geladenes Material für eine erwünschte Zeitdauer, so dass eine antimikrobielle Schicht-für-Schicht Beschichtung erhalten wird, die durch einen mittleren Kontaktwinkel von 80 Grad oder weniger **gekennzeichnet** ist, wobei das zweite geladene Material Ladungen aufweist, die den Ladungen des ersten geladenen Materials entgegengesetzt sind, wobei das erste geladene Material und das zweite geladene Material in einer solchen Menge vorhanden sind dass das Verhältnis der Ladungen des ersten geladenen Materials zu denen des zweiten geladenen Materials von 3:1 bis 100:1 ist, wobei mindestens eines des ersten und zweiten geladenen Materials ausgewählt ist aus der Gruppe bestehend aus geladenen antimikrobiellen Metall-Nanopartikeln; geladenen antimikrobiellen Metall-enthaltenden Nanopartikeln; Silber-Polyelektrolyt-Komplexe die aus Silberionen und einem polykationischen Material mit Aminogruppen gebildet sind; Silber-Polyelektrolyt-Komplexe die aus Silberionen und einem polyionischen Material mit Schwefel-enthaltende Gruppen gebildet sind; Silber-Nanopartikeln; und Kombinationen davon.

19. Verfahren gemäss Anspruch 18, wobei mindestens eines des ersten und zweiten geladenen Materials ausgewählt ist aus der Gruppe bestehend aus Silber-Polyelektrolyt-Komplexe die aus Silberionen und einem polykationischen Material mit Aminogruppen gebildet sind; Silber-Polyelektrolyt-Komplexe die aus Silberionen und einem polyionischen Material mit Schwefel-enthaltende Gruppen gebildet sind; und Kombinationen davon, und wobei das Verfahren ferner einen Schritt umfasst in dem die Silberionen in der antimikrobiellen Schicht-für-Schicht Beschichtung zu Silber-Nanopartikeln reduziert werden.

20. Verfahren gemäss Anspruch 19, wobei die Reduktion durch ein Reduktionsmittel, UV Strahlung oder Erwärmen erfolgt.

21. Verfahren gemäss Anspruch 19, umfassend, vor dem Schritt des Eintauchens, einen Schritt des vollständiges oder teilweisen Beschichtens der Oberfläche des Medizinalprodukts mit mindestens einem antimikrobiellen Mittel ausgewählt aus der Gruppe bestehend aus Polyquat welches antimikrobielle Aktivität zeigt, Furanonen, antimikrobiellen Peptiden, Isoxazolinonen, und organischen Selen-Verbindungen, wobei dieses mindestens eine antimikrobielle Mittel kovalent an die Oberfläche des Medizinalprodukts gebunden ist.

22. Verfahren gemäss Anspruch 18, ferner umfassend einen Schritt des kovalent Anbindens des mindestens einen antimikrobiellen Mittels an die antimikrobielle Metall-enthaltende Schicht-für-Schicht Beschichtung, durch die Reaktiven Gruppen der antimikrobiellen Metall-enthaltenden Schicht-für-Schicht Beschichtung, wobei das antimikrobielle Mittel ausgewählt ist aus der Gruppe bestehend aus Polyquat welches antimikrobielle Aktivität zeigt, Furanonen, antimikrobiellen Peptiden, Isoxazolinonen, und organischen Selen-Verbindungen.

23. Verfahren gemäss Anspruch 18, ferner umfassend einen Schritt des Behandeln des Medizinalprodukt mit einer antimikrobiellen Metall-enthaltenden Schicht-für-Schicht Beschichtung mittels Plasma zur Ausbildung einer Plasma-Beschichtung über der antimikrobiellen Metall-enthaltenden Schicht-für-Schicht Beschichtung.

24. Verfahren zur Herstellung eines Medizinalprodukts mit einer antimikrobiellen Metall-enthaltenden Schicht-für-Schicht Beschichtung darauf, wobei das Medizinalprodukt eine Kontaktlinse ist, umfassend die Schritte:
(a) Formen einer Form zur Herstellung der Kontaktlinse, wobei die Form einen ersten Formenteil umfasst der eine erste optische Oberfläche definiert und einen zweiten Formenteil der eine zweite optische Oberfläche definiert, wobei dieser erste Formenteil und dieser zweite Formenteil so ausgebildet sind, dass sie einander aufnehmen und dabei einen Kontaktlinsen-formenden Hohlraum zwischen dieser ersten optischen Oberfläche und der zweiten optischen Oberfläche bilden;
(b) Aufbringen einer übertragbaren antimikrobiellen Schicht-für-Schicht Beschichtung, mittels einer Schicht-für-Schicht Polyelektrolyt Ablagerungstechnik, auf mindestens eine der optischen Oberflächen, wobei die übertragbare antimikrobiellen Schicht-für-Schicht Beschichtung mindestens eine Schicht eines ersten geladenen Materials und mindestens eine Schicht eines zweiten geladenen Materials mit zu den Ladungen der ersten Schicht entgegengesetzten Ladungen umfasst, wobei mindestens eines der ersten und zweiten geladenen Materialien ausgewählt ist aus der Gruppe bestehend aus geladenen antimikrobiellen Metall-Nanopartikeln; geladenen antimikrobiellen Metall-enthaltenden Nanopartikeln; Silber-Polyelektrolyt-Komplexe die aus Silberionen und einem polykationischen Material mit Aminogruppen gebildet sind; Silber-Polyelektrolyt-Komplexe die aus Silberionen und einem polyionischen Material mit Schwefel-enthaltende Gruppen gebildet sind; Silber-Nanopartikeln; und Kombinationen davon;
(c) Fügen des ersten Formteils und des zweiten Formteils so dass die Formteile sich gegenseitig aufnehmen und die optischen Oberflächen den Kontaktlinsen-formenden Hohlraum bilden;
(d) Einfüllen einer polymerisierbaren Zusammensetzung in den Kontaktlinsen-formenden Hohlraum; und
(e) Härten der polymerisierbaren Zusammensetzung in dem Kontaktlinsen-formenden Hohlraum so dass die Kontaktlinse geformt wird, wobei die übertragbare antimikrobielle Schicht-für-Schicht Beschichtung sich von der mindestens einen optischen Oberfläche des Formteils ablöst und sich an die geformte Kontaktlinse anhaftet in der Art, dass die Kontaktlinse mit der antimikrobiellen Metall-enthaltenden Schicht-für-Schicht Beschichtung, die einen mittleren Kontaktwinkel von weniger als 80 Grad hat, beschichtet wird.

25. Verfahren gemäss Anspruch 24, wobei das Verfahren ferner einen Schritt umfasst in dem Silberionen der übertragbaren antimikrobiellen Schicht-für-Schicht Beschichtung oder der Silber-enthaltenden antimikrobiellen Schicht-für-Schicht Beschichtung zu Silber-Nanopartikeln reduziert werden.

26. Verfahren gemäss Anspruch 25, wobei die Reduktion durch ein Reduktionsmittel, UV Strahlung oder Erwärmen erfolgt.

27. Verfahren gemäss Anspruch 24, ferner umfassend einen Schritt des kovalent Anbindens des mindestens einen antimikrobiellen Mittels an die antimikrobielle Metall-enthaltende Schicht-für-Schicht Beschichtung, durch die Reaktiven Gruppen der antimikrobiellen Metall-enthaltenden Schicht-für-Schicht Beschichtung, wobei das antimikrobielle Mittel ausgewählt ist aus der Gruppe bestehend aus Polyquat welches antimikrobielle Aktivität zeigt, Furanonen, antimikrobiellen Peptiden, Isoxazolinonen, und organischen Selen-Verbindungen.

## Revendications

1. Dispositif médical comprenant un matériau d'âme et un revêtement couche par couche antimicrobien contenant du métal, dans lequel le revêtement couche par couche antimicrobien contenant du métal comprend un élément choisi dans le groupe constitué par: (a) une couche de nanoparticules de métal antimicrobiennes chargées; (b) une couche de nanoparticules antimicrobiennes chargées contenant du métal; (c) des complexes argent-polyélectrolyte formés entre des ions argent et un matériau polycationique ayant des groupes amino; (d) des complexes argent-polyélectrolyte formés entre des ions argent et un matériau polyionique ayant des groupes soufrés; (e) des nanoparticules d'argent; et (f) leurs combinaisons, dans lequel le revêtement couche par couche antimicrobien contenant du métal n'est pas fixé de manière covalente au matériau d'âme et confère au dispositif médical une hydrophilie **caractérisée par** le fait d'avoir un angle de contact moyen inférieur à 80 degrés.

2. Dispositif médical selon la revendication 1, ledit dispositif médical comprenant en outre un ou plusieurs agents antimicrobiens choisis dans le groupe constitué par les polyquats qui présentent une activité antimicrobienne, les furanones, les peptides antimicrobiens, les isoxazolinones et les composés organiques du sélénium.

3. Dispositif médical selon la revendication 2, dans lequel ledit ou lesdits agents antimicrobiens sont fixés de manière covalente à la surface du matériau d'âme.

4. Dispositif médical selon la revendication 2, dans lequel ledit ou lesdits agents antimicrobiens sont fixés de manière covalente au revêtement couche par couche antimicrobien contenant du métal par l'intermédiaire des sites réactifs du revêtement couche par couche antimicrobien contenant du métal.

5. Dispositif médical selon la revendication 1, dans lequel le revêtement couche par couche antimicrobien contenant du métal comprend une couche coiffante d'un matériau polyionique.

6. Dispositif médical selon la revendication 1, dans lequel le revêtement couche par couche antimicrobien contenant du métal comprend une bicouche d'électrolyte coiffante d'un matériau polyionique chargé positivement et un matériau polyionique chargé négativement ou une couche coiffante d'un matériau polymère chargé et d'un matériau polymère non chargé qui peut être fixé de manière non covalente au matériau polymère chargé.

7. Dispositif médical selon l'une quelconque des revendications 1 à 6, dans lequel le revêtement couche par couche antimicrobien comprend au moins une couche d'un polyquat antimicrobien.

8. Dispositif médical selon l'une quelconque des revendications 1 à 7, ledit dispositif médical étant une lentille de contact dure ou souple.

9. Lentille de contact selon la revendication 8, dans laquelle le revêtement couche par couche antimicrobien contenant du métal comprend: au moins une couche de nanoparticules de métal antimicrobiennes chargées et/ou de nanoparticules antimicrobiennes chargées contenant du métal; et au moins une couche d'un matériau polyionique ayant des charges opposées à celles des nanoparticules de métal antimicrobiennes chargées et/ou des nanoparticules antimicrobiennes chargées contenant du métal.

10. Lentille de contact selon la revendication 9, dans laquelle lesdites nanoparticules de métal antimicrobiennes chargées sont des nanoparticules d'argent chargées, et dans laquelle les nanoparticules antimicrobiennes chargées contenant du métal sont des nanoparticules chargées contenant de l'argent.

11. Lentille de contact selon la revendication 8, dans laquelle le revêtement couche par couche antimicrobien contenant du métal comprend au moins une couche d'un matériau polyionique chargé négativement et au moins une couche de complexes argent-polyélectrolyte formés entre des ions argent et un matériau polycationique ayant des groupes amino.

12. Lentille de contact selon la revendication 8, dans laquelle le revêtement couche par couche antimicrobien contenant du métal comprend au moins une couche d'un matériau polyionique chargé négativement et au moins une couche de complexes argent-polyélectrolyte formés entre des ions argent et un matériau polyionique ayant des groupes soufrés.

13. Lentille de contact selon la revendication 8, dans laquelle le revêtement couche par couche antimicrobien contenant du métal comprend au moins une couche d'un complexe argent-polyélectrolyte formé entre des ions argent et un matériau polyionique ayant des groupes soufrés.

14. Lentille de contact selon la revendication 8, dans laquelle le revêtement couche par couche antimicrobien contenant du métal comprend des nanoparticules d'argent.

15. Lentille de contact selon la revendication 14, dans laquelle les nanoparticules d'argent sont obtenues en formant tout d'abord un revêtement couche par couche de transition composé d'au moins une couche d'un premier matériau polyionique et d'au moins une couche d'un deuxième matériau polyionique ayant des charges opposées à celles du premier matériau polyionique, dans laquelle au moins l'un parmi le premier et le deuxième matériau polyionique est un complexe argent-polyélectrolyte formé entre des cations argent et un groupe amino chargé positivement, contenant un matériau polyionique, un complexe argent-polyélectrolyte formé entre des cations argent et un matériau polyionique ayant des groupes soufrés; et ensuite en réduisant les ions argent dans le revêtement couche par couche de transition au moyen d'un agent réducteur, d'une irradiation UV ou d'un chauffage.

16. Lentille de contact selon la revendication 8, ladite lentille de contact comprenant en outre un revêtement de plasma au-dessus du revêtement couche par couche antimicrobien contenant du métal.

17. Dispositif médical selon l'une quelconque des revendications 1 à 6, ledit dispositif médical étant une boîte ou un récipient qui sert à stocker un dispositif ophtalmique ou une solution ophtalmique.

18. Procédé de préparation d'un dispositif médical sur lequel se trouve un revêtement couche par couche antimicrobien contenant du métal, comprenant les étapes consistant à:
immerger ledit dispositif médical dans une solution contenant un premier matériau chargé et un deuxième matériau chargé pendant une période de temps désirée de manière à obtenir le revêtement couche par couche antimicrobien **caractérisé par** le fait d'avoir un angle de contact moyen de 80 degrés ou moins, dans lequel le deuxième matériau chargé a des charges opposées à celles du premier matériau chargé, dans lequel le premier matériau chargé et le deuxième matériau chargé sont présents en une quantité telle que le rapport des charges du premier matériau chargé au deuxième matériau chargé est de 3:1 à 100:1, dans lequel au moins l'un parmi le premier et le deuxième matériau chargé est choisi dans le groupe constitué par les nanoparticules de métal antimicrobiennes chargées, les nanoparticules antimicrobiennes chargées contenant du métal, les complexes argent-polyélectrolyte formés entre des ions argent et un matériau polycationique ayant des groupes amino, les complexes argent-polyélectrolyte formés entre des ions argent et un matériau polyionique ayant des groupes soufrés, et leurs combinaisons.

19. Procédé selon la revendication 18, dans lequel au moins l'un parmi le premier et le deuxième matériau chargé est choisi dans le groupe constitué par les complexes argent-polyélectrolyte formés entre des ions argent et un matériau polycationique ayant des groupes amino, les complexes argent-polyélectrolyte formés entre des ions argent et un matériau polyionique ayant des groupes soufrés, et leurs combinaisons, et ledit procédé comprenant en outre une étape de réduction des ions argent dans le revêtement couche par couche antimicrobien pour former des nanoparticules d'argent.

20. Procédé selon la revendication 19, dans lequel ladite réduction se fait au moyen d'un agent réducteur, d'une irradiation UV ou d'un chauffage.

21. Procédé selon la revendication 19, comprenant avant l'étape d'immersion, l'étape qui consiste à recouvrir complètement ou partiellement la surface du dispositif médical avec au moins un agent antimicrobien choisi dans le groupe constitué par un polyquat qui présente une activité antimicrobienne, les furanones, les peptides antimicrobiens, les isoxazolinones et les composés organiques du sélénium, où ledit au moins un agent antimicrobien est fixé de manière covalente à la surface du dispositif médical.

22. Procédé selon la revendication 18, comprenant en outre l'étape de fixation covalente d'au moins un agent antimicrobien au revêtement couche par couche antimicrobien contenant du métal par l'intermédiaire des sites réactifs du revêtement couche par couche antimicrobien contenant du métal, dans lequel ledit au moins un agent antimicrobien est choisi dans le groupe constitué par un polyquat qui présente une activité antimicrobienne, les furanones, les peptides antimicrobiens, les isoxazolinones et les composés organiques du sélénium.

23. Procédé selon la revendication 18, comprenant en outre l'étape consistant à soumettre le dispositif médical ayant le revêtement couche par couche antimicrobien contenant du métal à un traitement par plasma pour former un revêtement de plasma au-dessus du revêtement couche par couche antimicrobien contenant du métal.

24. Procédé de production d'un dispositif médical sur lequel se trouve un revêtement couche par couche antimicrobien contenant du métal, dans lequel le dispositif médical est une lentille de contact, comprenant les étapes consistant à:
(a) former un moule pour fabriquer la lentille de contact, où le moule comprend une première partie de moule définissant une première surface optique et une deuxième partie de moule définissant une deuxième surface optique, dans lequel ladite première partie de moule et ladite deuxième partie de moule sont configurées pour s'imbriquer l'une à l'autre de sorte qu'il se forme entre ladite première surface optique et ladite deuxième surface optique une cavité formant la lentille de contact;
(b) appliquer un revêtement couche par couche antimicrobien transférable en utilisant une technique de dépôt de polyélectrolyte couche par couche sur au moins une desdites surfaces optiques, où le revêtement couche par couche antimicrobien transférable comprend au moins une couche d'un premier matériau chargé et au moins une couche d'un deuxième matériau chargé ayant des charges opposées à celles du premier matériau chargé, où au moins l'un parmi le premier et le deuxième matériau chargé est choisi dans le groupe constitué par les nanoparticules de métal antimicrobiennes chargées, les nanoparticules antimicrobiennes chargées contenant du métal, les complexes argent-polyélectrolyte formés entre les ions argent et un matériau polycationique ayant des groupes amino, les complexes argent-polyélectrolyte formés entre les ions argent et un matériau polyionique ayant des groupes soufrés, et leurs combinaisons;
(c) positionner ladite première partie de moule et ladite deuxième partie de moule de telle façon que lesdites parties de moule s'imbriquent l'une à l'autre et que lesdites surfaces optiques définissent ladite cavité formant la lentille de contact;
(d) introduire une composition polymérisable dans ladite cavité formant la lentille de contact; et
(e) durcir ladite composition polymérisable à l'intérieur de ladite cavité formant la lentille de contact de façon à former la lentille de contact, ce qui fait que ledit revêtement couche par couche antimicrobien transférable se détache de ladite au moins une surface optique de ladite partie du moule et se fixe à nouveau à ladite lentille de contact formée de sorte que ladite lentille de contact est recouverte du revêtement couche par couche antimicrobien contenant du métal, ayant un angle de contact moyen inférieur à 80 degrés.

25. Procédé selon la revendication 24, ledit procédé comprenant en outre une étape de réduction des ions argent dans le revêtement couche par couche antimicrobien transférable ou dans le revêtement couche par couche antimicrobien contenant de l'argent pour former des nanoparticules d'argent.

26. Procédé selon la revendication 25, dans lequel ladite réduction se fait au moyen d'un agent réducteur, d'une irradiation UV ou d'un chauffage.

27. Procédé selon la revendication 24, comprenant en outre l'étape de fixation covalente d'au moins un agent antimicrobien au revêtement couche par couche antimicrobien par l'intermédiaire des sites réactifs du revêtement couche par couche antimicrobien sur la lentille de contact, où ledit au moins un agent antimicrobien est choisi dans le groupe constitué par un polyquat qui présente une activité antimicrobienne, les furanones, les peptides antimicrobiens, les isoxazolinones et les composés organiques du sélénium.
